# EUROPEAN PATENT SPECIFICATION

(11) **EP 3 130 582 B1**
(45) Date of publication and mention of the grant of the patent: **10.10.2018**
(21) Application number: 15786179.0
(22) Date of filing: 29.04.2015
(51) Int. Cl.: C07C 279/26, A61K 31/155, A61P 37/00

(54) **COMPOUND HAVING IMMUNE DISEASE TREATMENT EFFECT AND USE THEREOF**
VERBINDUNG MIT IMMUNERKRANKUNGBEHANDLUNGSWIRKUNG UND VERWENDUNG DAVON
COMPOSÉ AYANT UN EFFET THÉRAPEUTIQUE SUR LES MALADIES IMMUNITAIRES ET UTILISATION DE CE COMPOSÉ

(30) Priority: 29.04.2014 KR 20140051261
(43) Date of publication of application: 15.02.2017
(73) Proprietor: The Catholic University Of Korea Industry-Academic, Seoul 137-701 (KR)
(72) Inventor: CHO, Mi-La, Seoul 140-854 (KR); SHIN, Dong-Yun, Seoul 135-906 (KR); PARK, Sung-Hwan, Seoul 137-779 (KR); YANG, Chul- Woo, Seoul 137-764 (KR); CHOI, Jong-Young, Seoul 137-855 (KR); PARK, Min-Jung, Seoul 152-781 (KR); SON, Hye-Jin, Seoul 150-833 (KR); LEE, Sung-Hee, Seoul 156-803 (KR); LEE, Seon-Yeong, Seoul 157-883 (KR); KIM, Eun-Kyung, Seoul 139-937 (KR); KIM, Jae-kyung, Seoul 151-849 (KR); LEE, Seung-Hun, Goyang-si Gyeonggi-do 10412 (KR); PARK, Seong-Hyeok, Yeongju-si Gyeongsangbuk-do 36132 (KR)
(74) Representative: Turner, Craig Robert
(86) International application number: PCT/KR2015/004299
(87) International publication number: WO 2015/167243

(56) References cited:
- WO-A2-03/084475
- WO-A2-03/089601
- KR-A- 20110 053 837
- KR-A- 20110 081 095
- KR-A- 20140 036 598
- US-A- 4 060 635
- US-A- 4 183 956
- US-A1- 2011 053 941
- US-A1- 2013 095 140
- H. C. CARRINGTON ET AL: "Synthetic antimalarials. Part XLIX. The structure and synthesis of the dihydrotriazine metabolite of proguanil", JOURNAL OF THE CHEMICAL SOCIETY (RESUMED), 1 January 1954 (1954-01-01), pages 1017-1031, XP055061878, ISSN: 0368-1769, DOI: 10.1039/jr9540001017
- SHAPIRO S L ET AL: "HYPOGLYCEMIC AGENTS. II. ARYLBIGUANIDES", JOURNAL OF THE AMERICAN CHEMICAL SOCIETY, vol. 81, 20 July 1959 (1959-07-20), pages 3725-3728, XP001160743, AMERICAN CHEMICAL SOCIETY, US ISSN: 0002-7863, DOI: 10.1021/JA01523A059
- DATABASE PubChem Compound [Online] 20 October 2014 (2014-10-20), XP055390088, Database accession no. AKOS021352901
- DATABASE PubChem Compound [Online] 20 October 2014 (2014-10-20), XP055390098, Database accession no. AKOS021352899
- DATABASE PubChem Compound [Online] 30 November 2012 (2012-11-30), XP055390103, Database accession no. CID 68132850
- RICHARD A. GLENNON ET AL: "Arylguanidine and arylbiguanide binding at 5-HT3 serotonin receptors: A QSAR study", BIOORGANIC & MEDICINAL CHEMISTRY, vol. 11, no. 20, 1 October 2003 (2003-10-01), pages 4449-4454, XP055390154, GB ISSN: 0968-0896, DOI: 10.1016/S0968-0896(03)00488-7
- CURD F H S ET AL: "SYNTHETIC ANTIMALARIALS. PART IV. 2-PHENYLGUANIDINO-4-AMINOALKYLAMINO-6-METH YLPYRIMIDINES", JOURNAL OF THE CHEMICAL SOC, 1 January 1946 (1946-01-01), pages 362-366, XP009022670, CHEMICAL SOCIETY, LETCHWORTH; GB ISSN: 0368-1769, DOI: 10.1039/JR9460000362
- DATABASE REGISTRY [Online] CHEMICAL ABSTRACTS SERVICE, COLUMBUS, OHIO, US; 25 February 2014 (2014-02-25), XP002772055, Database accession no. 1555308-85-4

## Description

### TECHNICAL FIELD

The present invention relates to a novel compound having excellent treating and preventing effects of immune diseases and a use thereof.

### BACKGROUND ART

Immune diseases are the diseases that the components of the mammalian immune system cause, mediate or contribute to pathological states of mammals. In particular, inflammatory disorders are one of the most important health problems in the world. Generally, inflammation is a localized protective response of the body tissues against the host invasion by foreign substances or harmful stimuli. The causes of inflammation includes infections such as bacterial infection, viral infection, or parasitic infection; physical causes such as burns or irradiation; chemicals such as toxins, drugs or industrial agents; immunological response such as allergic and autoimmune reactions; or conditions associated with oxidative stress.

Inflammation is characterized by pain, redness, swelling, fever, and ultimate loss of function of the affected sites. These symptoms are the result of a complex series of interactions between cells of the immune system. The responses between the cells result in the interaction network of various groups of inflammatory mediators: proteins (e.g., cytokines, enzymes (e.g. protease, peroxidase), major basic proteins, adhesion molecules (ICAM, VCAM), lipid mediators (e.g., eicosanoids, prostaglandins, leukotrienes, platelet activating factor (PAF)), reactive oxygen species (e.g., hydroperoxide, superoxide anion O2-, nitric oxide (NO), and the like). However, most of these inflammatory mediators are also the regulators in normal cellular activities; and thus in case of deficiency of inflammatory reaction, the host cannot control damages (i.e., infections). Chronic inflammation causes the inflammatory diseases mediated by excessive formation of many of the above-mentioned mediators.

And also, autoimmune disease, one of immune diseases, is characterized by spontaneous responses of the immune system to attack one's own organs. These responses are caused by the autoantigen recognition by T lymphocytes, thereby inducing humoral immune responses (autoantigen generated) and cell-mediated immune responses (the cytotoxic activities of lymphocytes and macrophages increased). The autoimmune diseases include the followings: rheumatoid diseases, psoriasis, systemic dermatomyositis, multiple sclerosis, lupus erythematosus, or deterioration of the immune response to antigens, i.e., asthma, drug or food allergy or the like. All of these diseases are restrictive and chronic diseases; and also are fatal in some cases. However, it is current situation that there is no effective therapy for treating said diseases so far. Therefore, it is expected that drugs, medicines, or agents capable of relieving or alleviating the progression of such diseases will be important means for the patient's health.

Various researches on the therapies of autoimmune diseases have been performed in order to find out appropriate drugs and method for the treatment thereof. The current therapies of autoimmune diseases are mainly based on the uses of immunosuppressive drugs such as glucocorticoids, calcineurin inhibitors, and antiproliferatives-antimetabolites - see document WO 2003/089601, which relates to inhibition of 2-deoxyglucosone; US 2013/095140, which relates to treatment of metabolic disorders; and US 2011/053941, which relates to indoleamine 2,3-dioxygenase mediated immunosuppression However, these pharmacological therapies act on a variety of targets, which may cause lowering the overall immune function. On the other hand, a long-term use of these pharmacological therapies causes various problems of cytotoxicity and suppresses the immune system non-specifically, which can make the patients exposed to the risk of infections and cancers. Since calcineurin inhibitors and glucocorticoids bring about another problem due to their nephrotoxicity and diabetes-inducing properties, the use thereof is limited in the cases of some clinical symptoms (such as renal insufficiency, diabetes, etc.).

Therefore, there is a need to develop novel therapeutic agents for treating immune diseases such as autoimmune diseases and inflammatory diseases, which have excellent therapeutic effects without side effects.The present inventors have carried out researches to find out the agents that can effectively prevent or treat immune diseases and show low side effects; and, as results
thereof, confirmed that the synthesized compounds can effectively treat immune diseases; and completed the present invention.

N-ethyl-N-(4-fluorophenyl)-biguanide (RN 1555308-85-4) has been disclosed in the chemical catalog of Aurora Fine Chemicals. In the following it is also called compound SD-282.

### DISCLOSURE

### Technical Problem

Therefore, it is an object of the present disclosure to provide a compound.

It is an object of the present invention to provide a pharmaceutical composition for preventing or treating an immune disease, comprising the compound as an active ingredient.

In addition, it is still another object of the present disclosure to provide an immunomodulator comprising the compound as an active ingredient.

In addition, it is still another object of the present disclosure to provide a method for reducing the differentiation of native T cells to Th17 cells and the activity of Th17 cells, which comprises treating native T cells with the compound *in vitro.*

In addition, it is still another object of the present disclosure to provide a method for increasing the differentiation of native T cells to Treg cells and the activity of Treg cells, which comprises treating native T cells with the compound *in vitro.*

### Technical Solution

The present invention relates to a pharmaceutical composition for use in facilitating or increasing the activity of regulatory T cells and reducing or inhibiting the activity of pathogenic Th17 cells in an immune disease, comprising N- ethyl- N- (4- fluorophenyl)- biguanide or a pharmaceutically acceptable salt thereof.

In an embodiment, the compound may reduce or inhibit proinflammatory cytokine formation, inhibit autoantibody production, and inhibit differentiation to osteoclasts.

In an embodiment, the proinflammatory cytokine may be IL-17, IL-6, TNF-α, IFN-γ, MMP-9 or STAT-3.

In an embodiment, the antibody may be IgG, IgG1 or IgG2a.

In an embodiment, the composition may comprise the compound in the concentration ranging from 0.1 mM to 10 mM.

In an embodiment, the immune disease may be selected from the group consisting of an autoimmune disease; an inflammatory disease; and a transplantation rejection disease of cells, tissues or organs.

In an embodiment, the immune disease may be selected from rheumatoid arthritis, Behcet's disease, polymyositis or dermatomyositis, autoimmune cytopenia, autoimmune myocarditis, atopic dermatitis, asthma, primary cirrhosis, dermatomyositis, Goodpasture's syndrome, autoimmune meningitis, Sjogren's syndrome, lupus, Addison's disease, alopecia areata, ankylosing myelitis, autoimmune hepatitis, autoimmune mumps, Crohn's disease, insulin-dependent diabetes, dystrophic epidermolysis bullosa, epididymitis, glomerulonephritis, Graves' disease, Guillain-Barre syndrome, Hashimoto's disease, hemolytic anemia, multiple sclerosis, myasthenia gravis, pemphigus vulgaris, psoriasis, rheumatic fever, sarcoidosis, scleroderma, spondyloarthropathy, thyroiditis, vasculitis, vitiligo, myxedema, pernicious anemia, mitochondria-related syndrome and ulcerative colitis.

In an embodiment, the transplantation rejection disease may be graft versus host disease.

In addition, the present disclosure provides an immunomodulator comprising the compound as an active ingredient.

In addition, the present disclosure provides a method for reducing the differentiation of native T cells to Th17 cells and the activity of Th17 cells, which comprises treating native T cells with the compound *in vitro.*

In addition, the present disclosure provides a method for increasing the differentiation of native T cells to Treg cells and the activity of Treg cells, which comprises treating native T cells with the compound *in vitro.*

### ADVANTAGEOUS EFFECTS

The present invention relates to the compound SD-282 capable of effectively preventing and treating immune diseases and a use thereof. The compound SD-282 has effects of inhibiting the formation of proinflammatory cytokines, increasing the activity of regulatory T cells having immunoregulatory functions, inhibiting the production of autoantibodies to regulate excessive immune responses, and inhibiting the differentiation to osteoclasts, and thus can be used for treating immune diseases, such as autoimmune diseases, inflammatory diseases, and transplant rejection diseases, which are caused by abnormal regulation of various kinds of immune responses.

### DESCRIPTION OF DRAWINGS

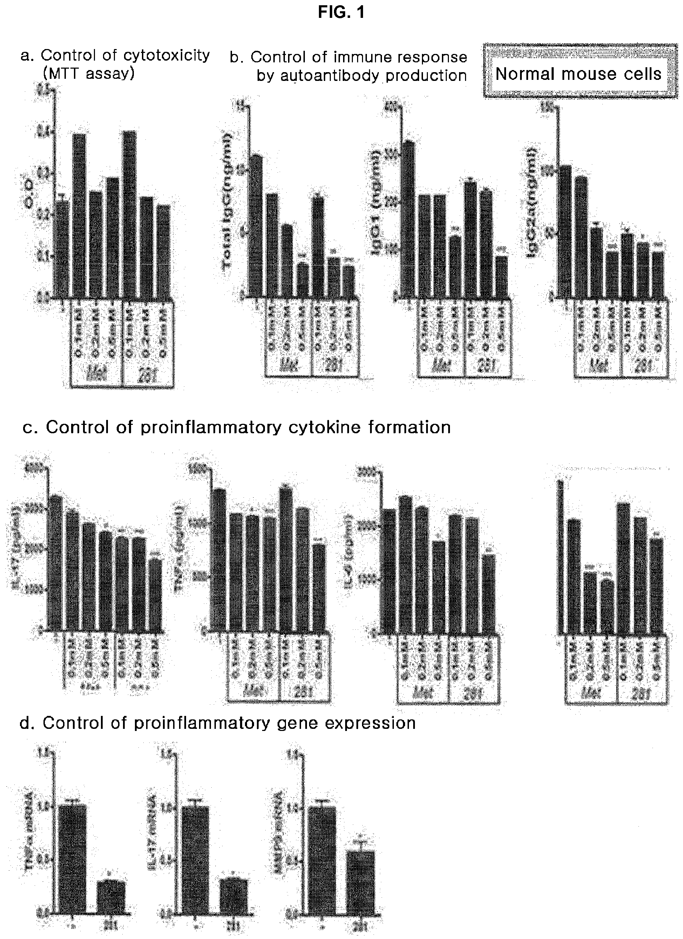

FIG. 1 shows the results obtained by analyzing cytotoxicity, autoantibody production, formation of proinflammatory cytokines, and expression of proinflammatory genes, according to treating the splenocytes derived from normal mouse with the compound SD-281 in predetermined concentrations. Whether the differentiation to osteoclasts is regulated according to treating with the compound SD-281 was also observed.

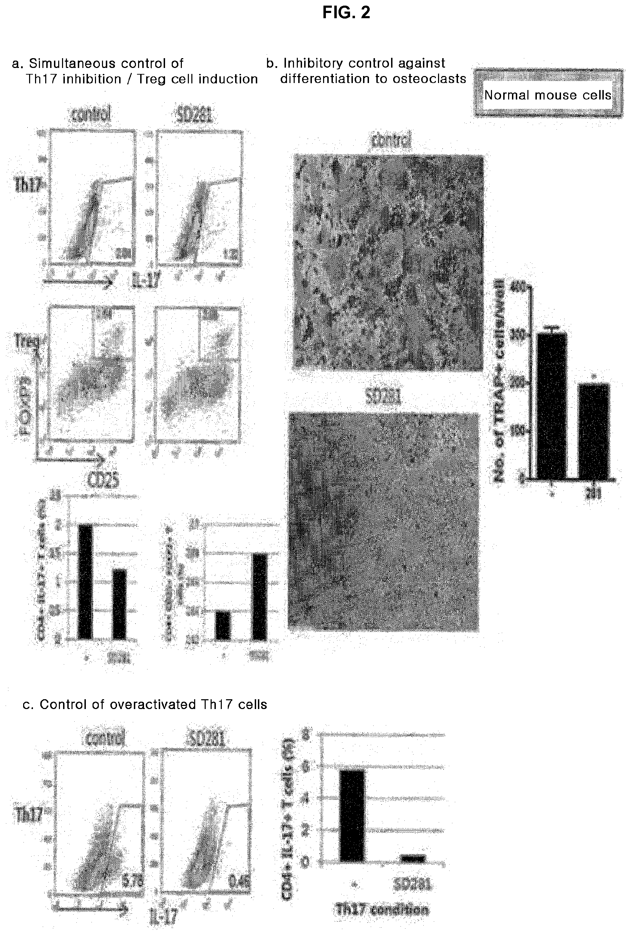

FIG. 2 shows the results obtained by analyzing Th17 inhibition & Treg activity facilitation; and inhibition of overactivated Th17, according to treating the splenocytes derived from normal mouse with the compound SD-281 in predetermined concentrations; and the results obtained by analyzing inhibitory ability against differentiation of mouse bone marrow (BM) cells to osteoclasts, according to treating with the compound SD-281.

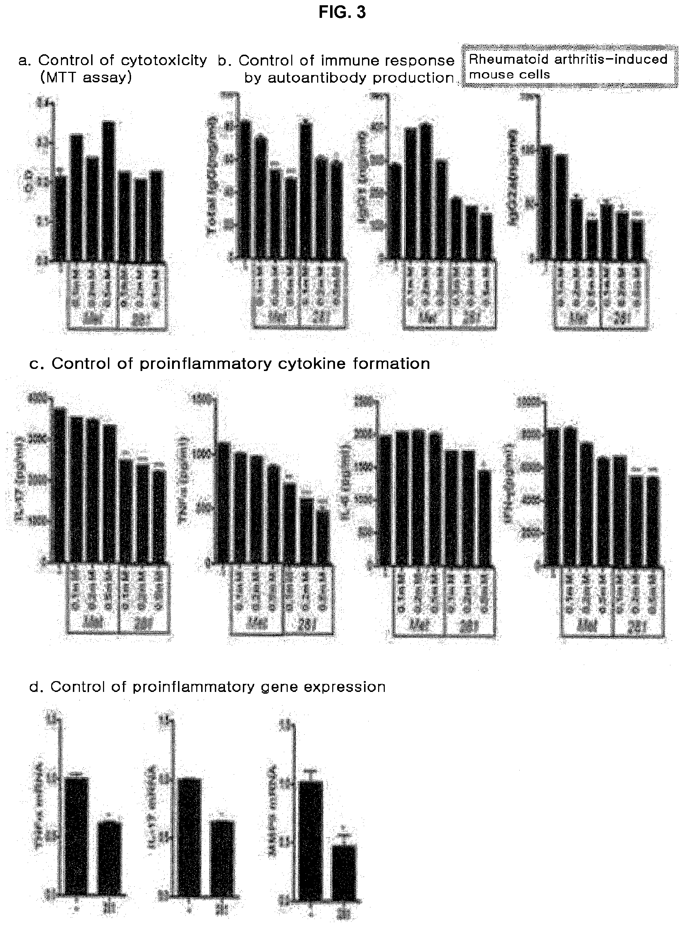

FIG. 3 shows the results obtained by analyzing cytotoxicity, autoantibody production, formation of proinflammatory cytokines, and expression of proinflammatory genes, according to treating the splenocytes derived from rheumatoid arthritis-induced mouse with the compound SD-281 in predetermined concentrations.

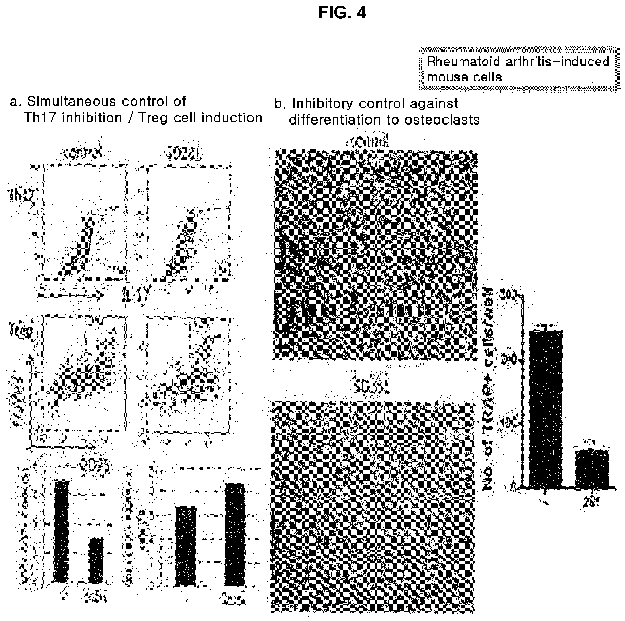

FIG. 4 shows the results obtained by analyzing Th17 inhibition & Treg activity facilitation; and inhibition of the differentiation to osteoclasts, according to treating the splenocytes derived from rheumatoid arthritis-induced mouse with the compound SD-281 in predetermined concentrations.

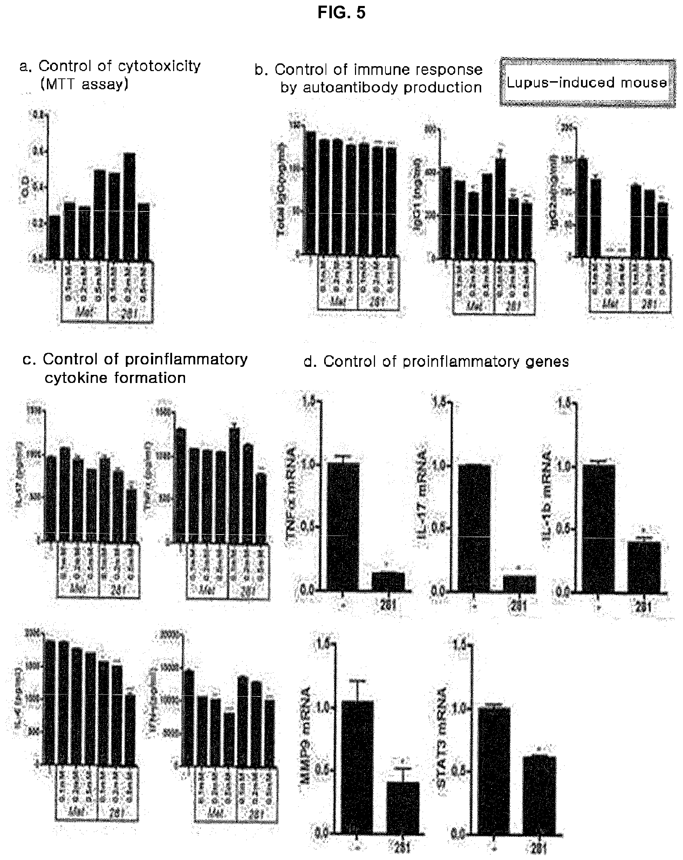

FIG. 5 shows the results obtained by analyzing cytotoxicity, autoantibody production, formation of proinflammatory cytokines, and expression of proinflammatory genes, according to treating the splenocytes derived from lupus-induced mouse with the compound SD-281 in predetermined concentrations.

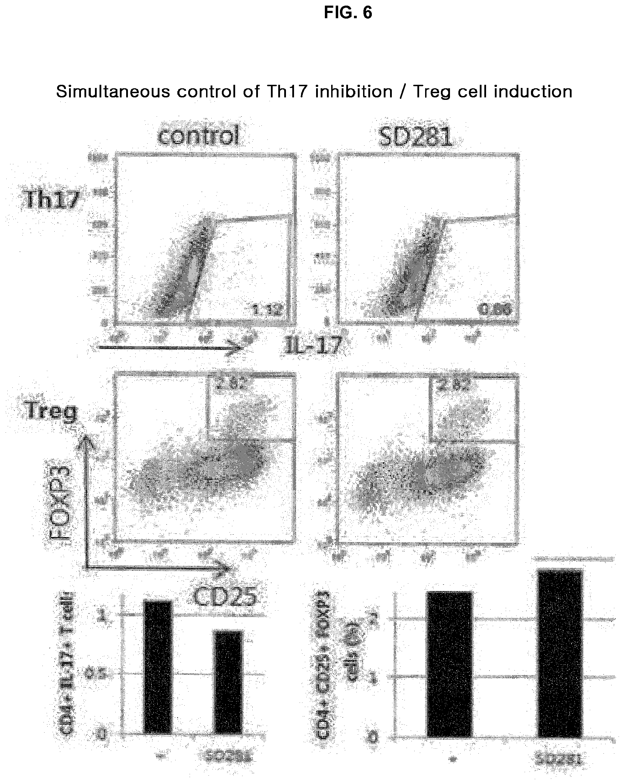

FIG. 6 shows the results obtained by analyzing Th17 inhibition & Treg activity facilitation, according to treating the splenocytes derived from lupus-induced mouse with the compound SD-281 in predetermined concentrations.

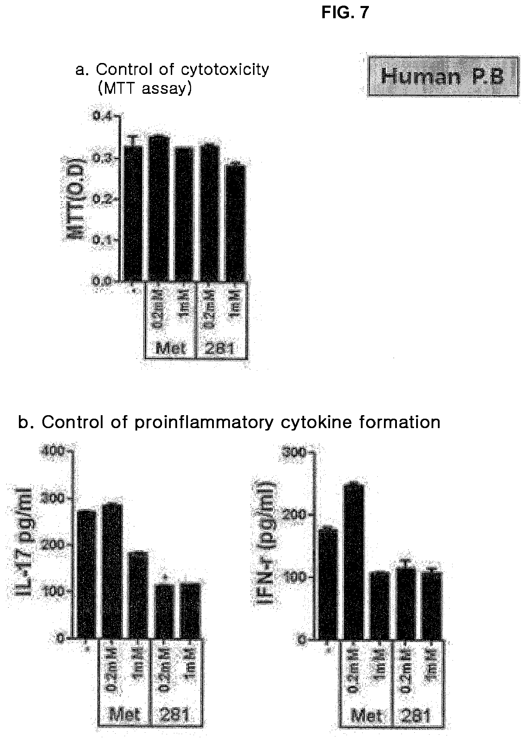

FIG. 7 shows the results obtained by analyzing cytotoxicity and formation of proinflammatory cytokines, according to treating the lymphocytes derived from human peripheral blood with the compound SD-281 in predetermined concentrations.

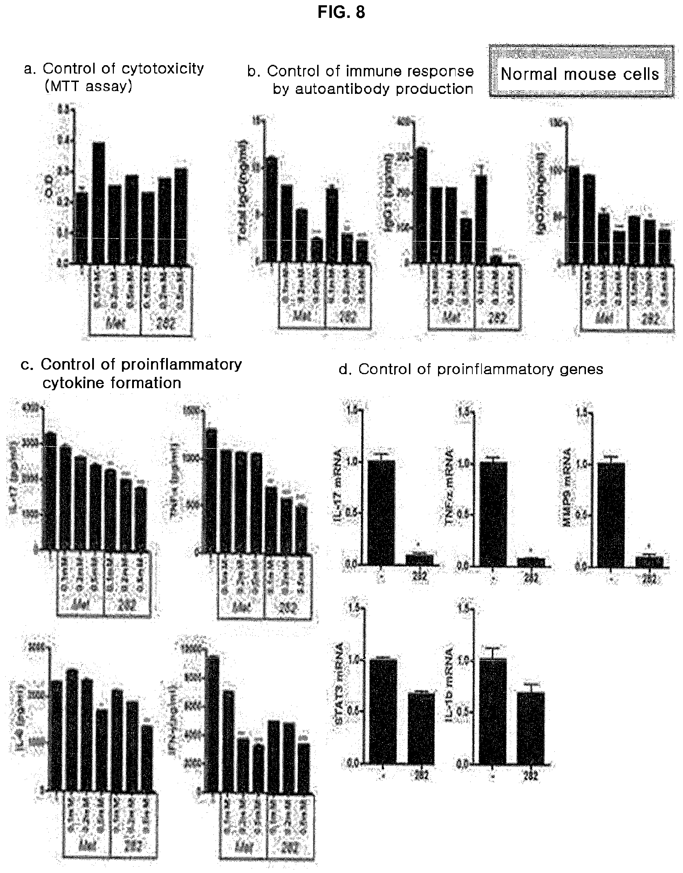

FIG. 8 shows the results obtained by analyzing cytotoxicity, autoantibody production, formation of proinflammatory cytokines, and expression of proinflammatory genes, according to treating the splenocytes derived from normal mouse with the compound SD-282 in predetermined concentrations.

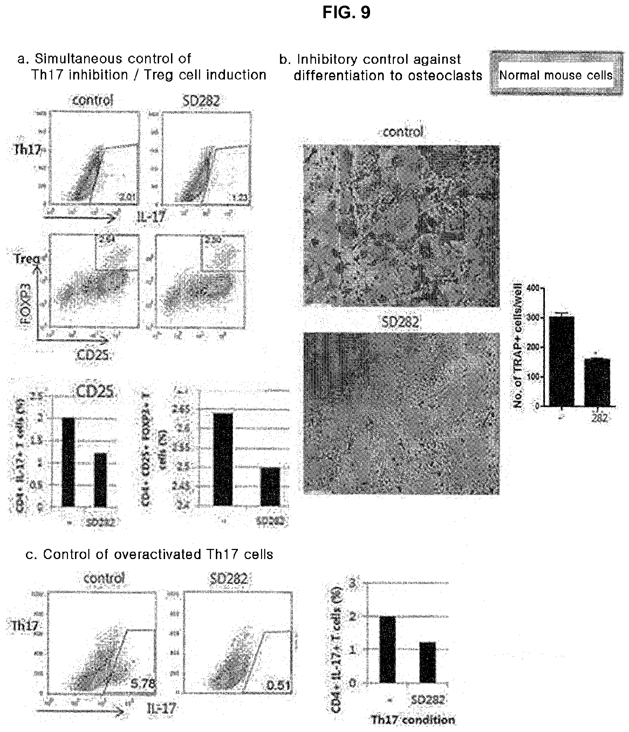

FIG. 9 shows the results obtained by analyzing Th17 inhibition & Treg activity facilitation, inhibition of the differentiation to osteoclasts, and inhibition of overactivated Th17, according to treating the splenocytes derived from normal mouse with the compound SD-282 in predetermined concentrations.

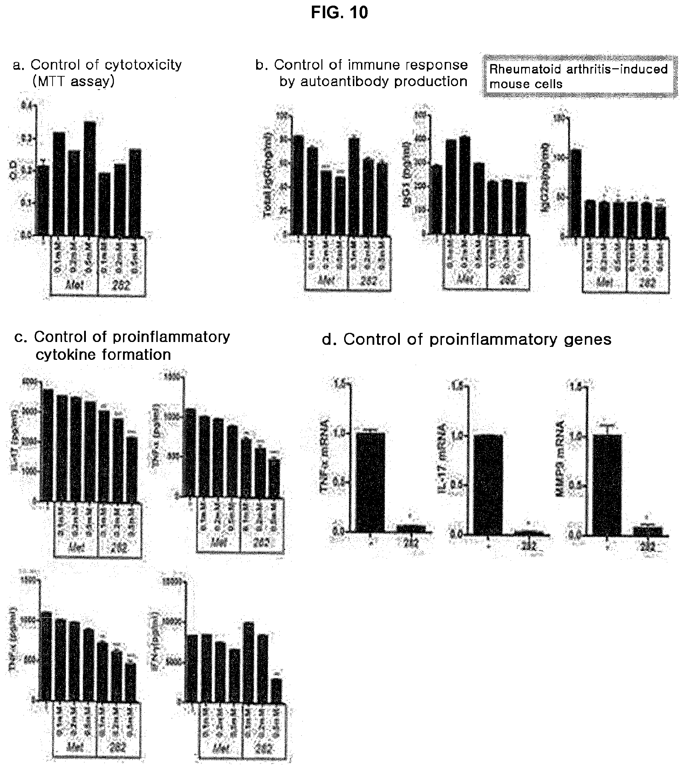

FIG. 10 shows the results obtained by analyzing cytotoxicity, autoantibody production, formation of proinflammatory cytokines, and expression of proinflammatory genes, according to treating the splenocytes derived from rheumatoid arthritis-induced mouse with the compound SD-282 in predetermined concentrations.

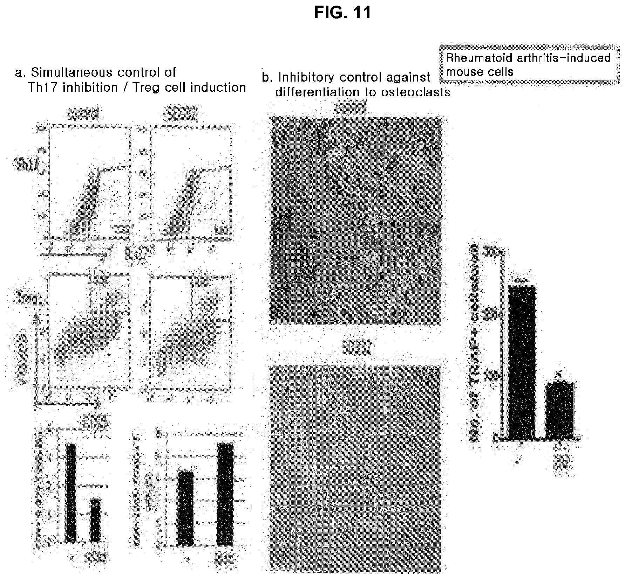

FIG. 11 shows the results obtained by analyzing Th17 inhibition & Treg activity facilitation; and inhibition of the differentiation to osteoclasts, according to treating the splenocytes derived from rheumatoid arthritis-induced mouse with the compound SD-282 in predetermined concentrations.

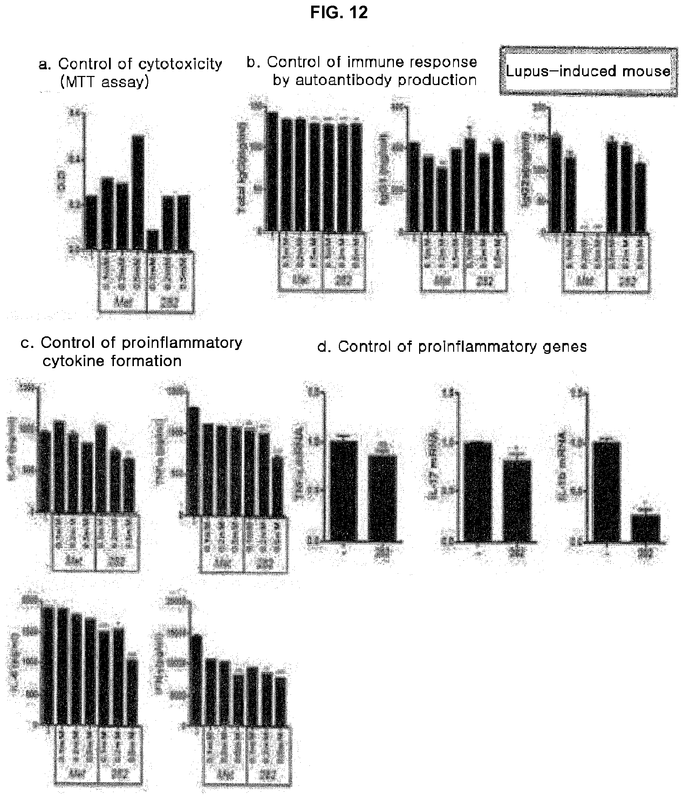

FIG. 12 shows the results obtained by analyzing cytotoxicity, autoantibody production, formation of proinflammatory cytokines, and expression of proinflammatory genes, according to treating the splenocytes derived from lupus-induced mouse with the compound SD-282 in predetermined concentrations.

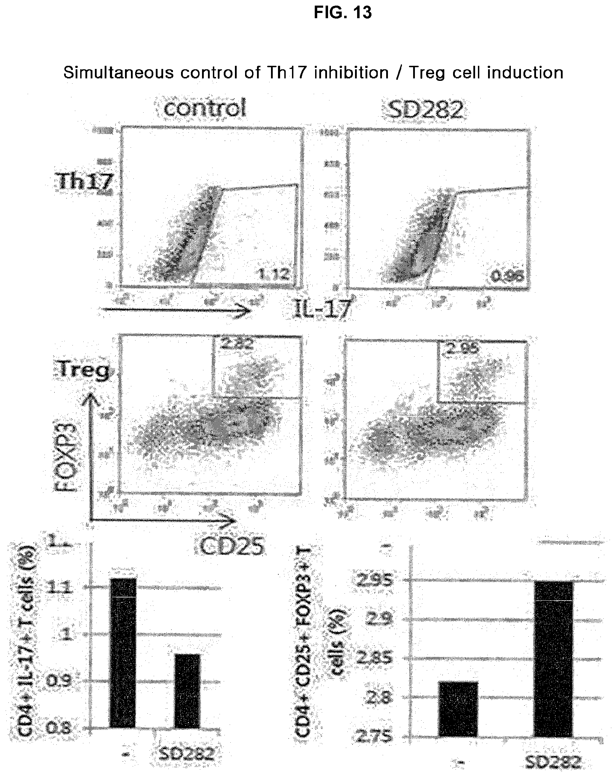

FIG. 13 shows the results obtained by analyzing Th17 inhibition & Treg activity facilitation, according to treating the splenocytes derived from lupus-induced mouse with the compound SD-282 in predetermined concentrations.

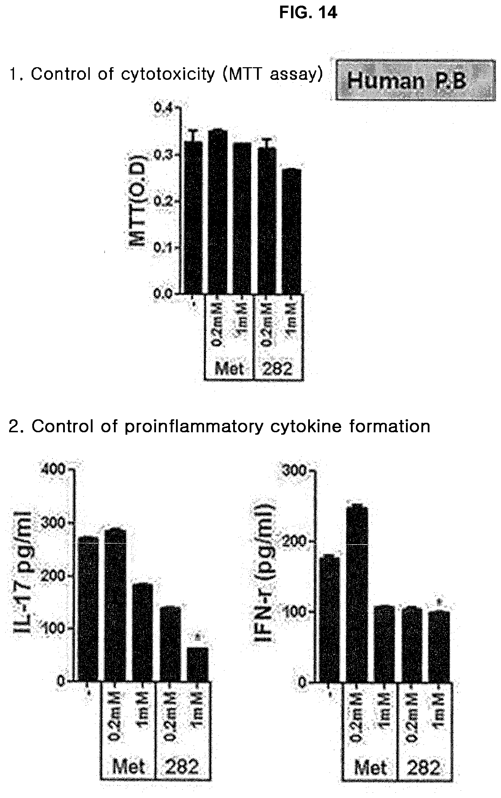

FIG. 14 shows the results obtained by analyzing cytotoxicity and formation of proinflammatory cytokines, according to treating the lymphocytes derived from human peripheral blood with the compound SD-282 in predetermined concentrations.

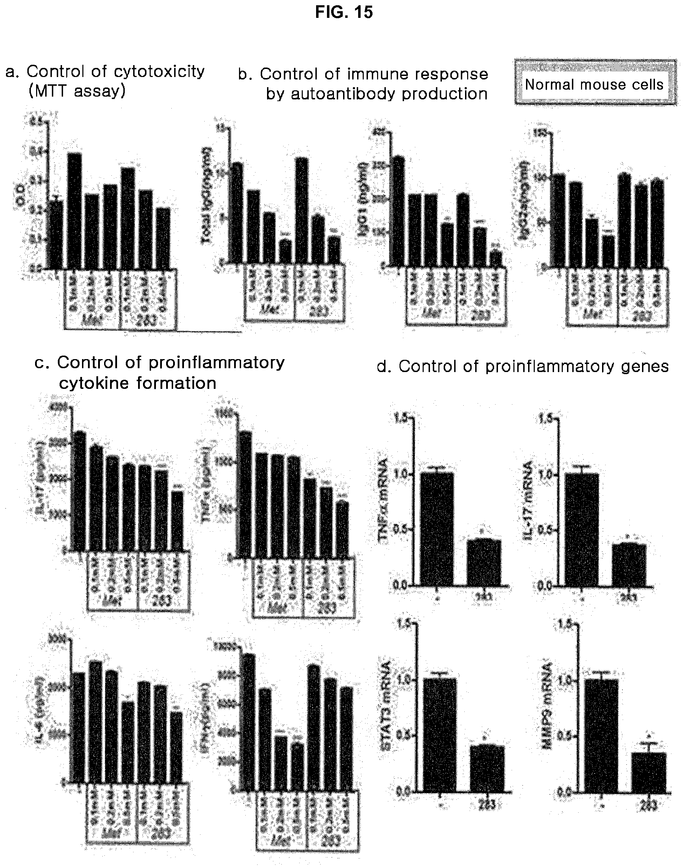

FIG. 15 shows the results obtained by analyzing cytotoxicity, autoantibody production, formation of proinflammatory cytokines, and expression of proinflammatory genes, according to treating the splenocytes derived from normal mouse with the compound SD-283 in predetermined concentrations.

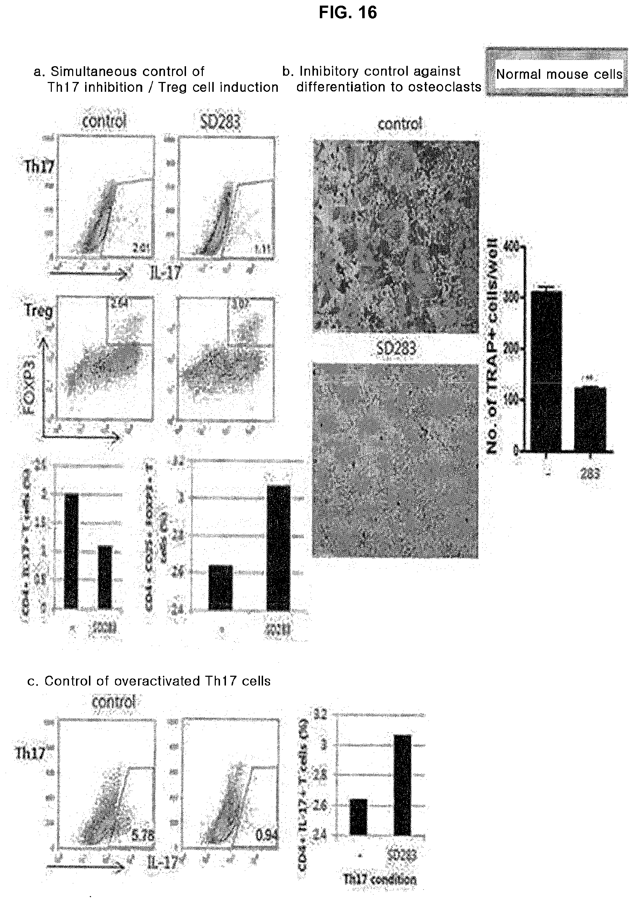

FIG. 16 shows the results obtained by analyzing Th17 inhibition & Treg activity facilitation, inhibition of the differentiation to osteoclasts, and inhibition of overactivated Th17, according to treating the splenocytes derived from normal mouse with the compound SD-283 in predetermined concentrations.

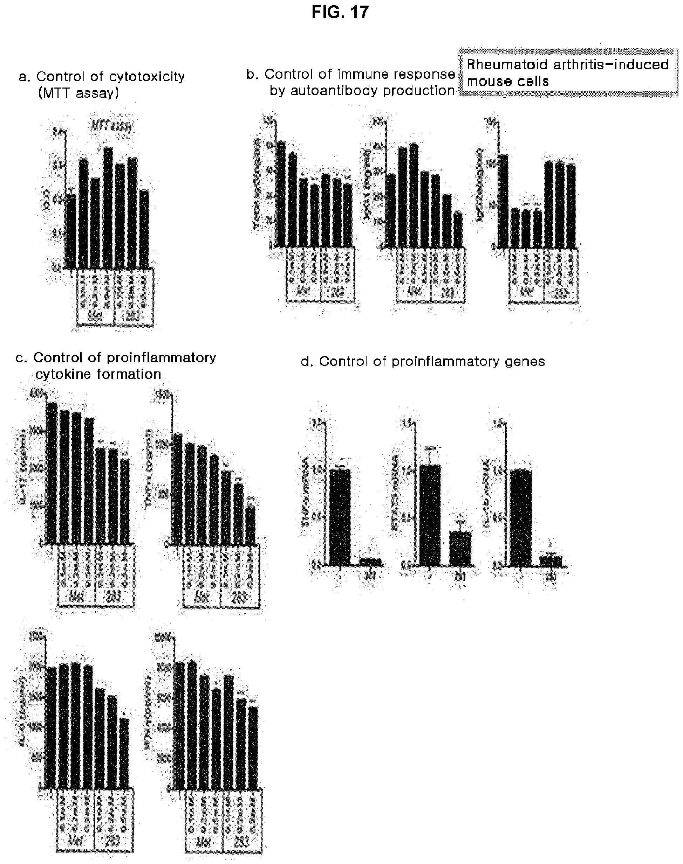

FIG. 17 shows the results obtained by analyzing cytotoxicity, autoantibody production, formation of proinflammatory cytokines, and expression of proinflammatory genes, according to treating the splenocytes derived from rheumatoid arthritis-induced mouse with the compound SD-283 in predetermined concentrations.

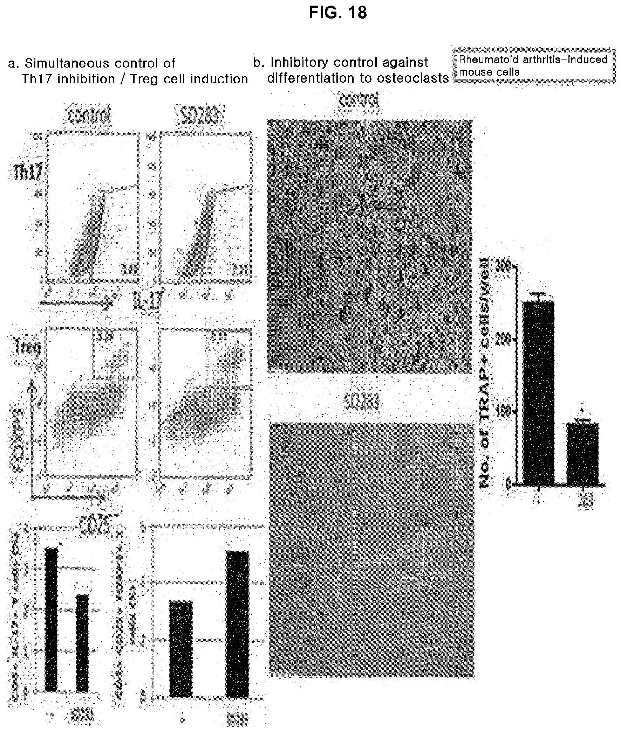

FIG. 18 shows the results obtained by analyzing Th17 inhibition & Treg activity facilitation; and inhibition of the differentiation to osteoclasts, according to treating the splenocytes derived from rheumatoid arthritis-induced mouse with the compound SD-283 in predetermined concentrations.

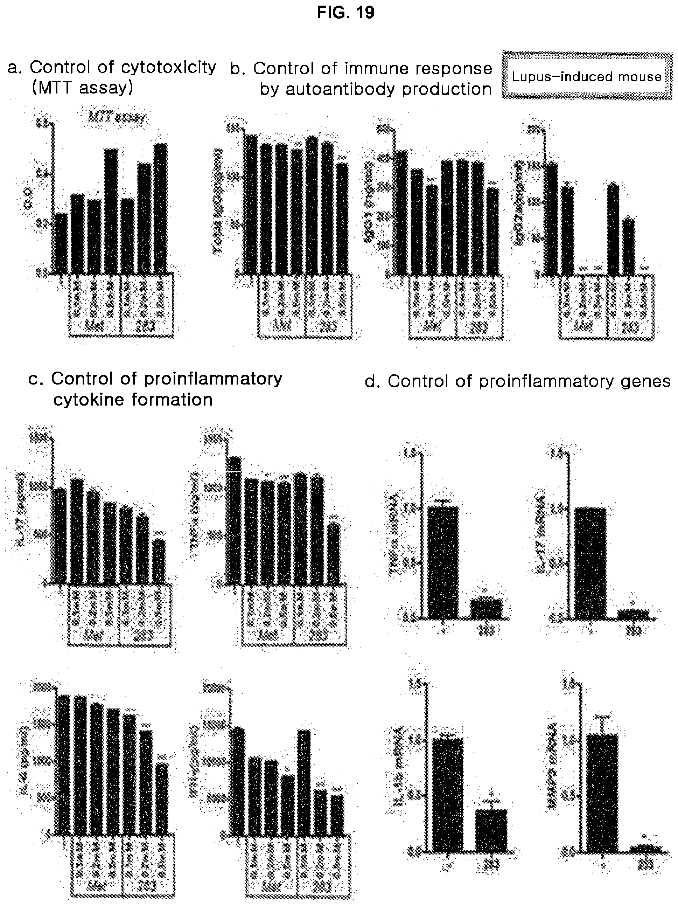

FIG. 19 shows the results obtained by analyzing cytotoxicity, autoantibody production, formation of proinflammatory cytokines, and expression of proinflammatory genes, according to treating the splenocytes derived from lupus-induced mouse with the compound SD-283 in predetermined concentrations.

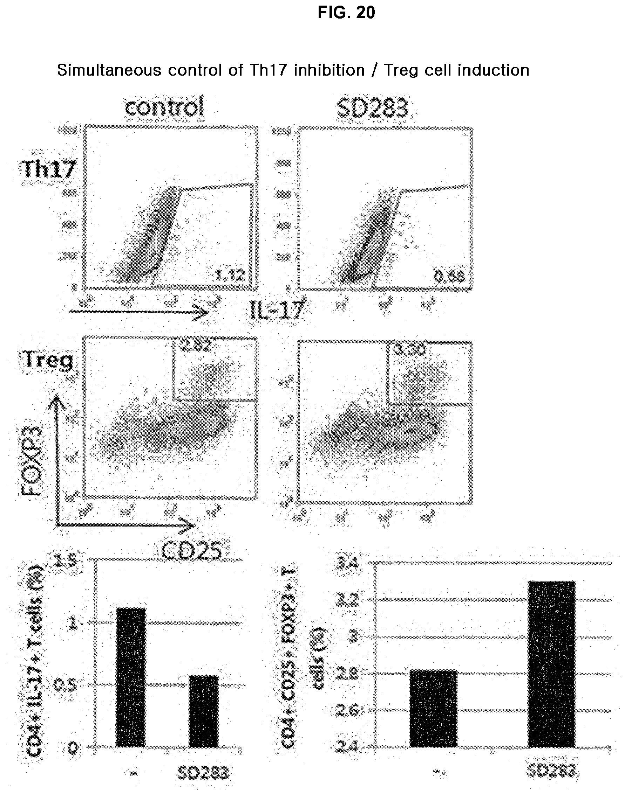

FIG. 20 shows the results obtained by analyzing Th17 inhibition & Treg activity facilitation, according to treating the splenocytes derived from lupus-induced mouse with the compound SD-283 in predetermined concentrations.

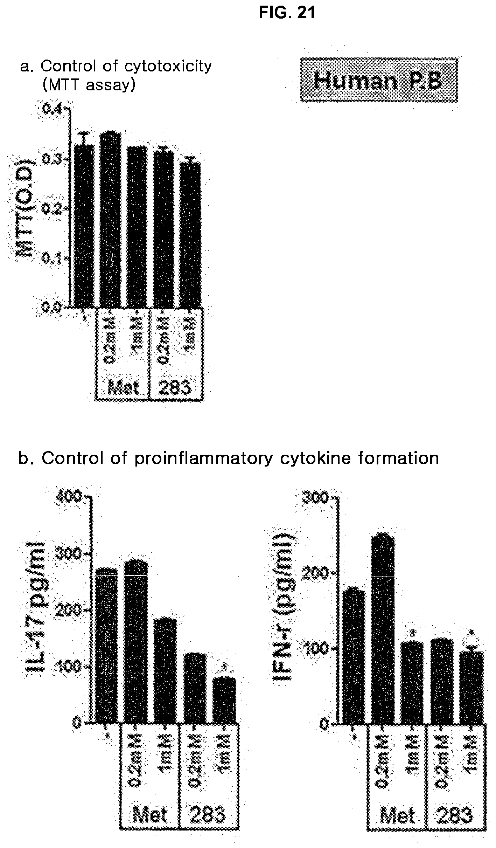

FIG. 21 shows the results obtained by analyzing cytotoxicity and formation of proinflammatory cytokines, according to treating the lymphocytes derived from human peripheral blood with the compound SD-283 in predetermined concentrations.

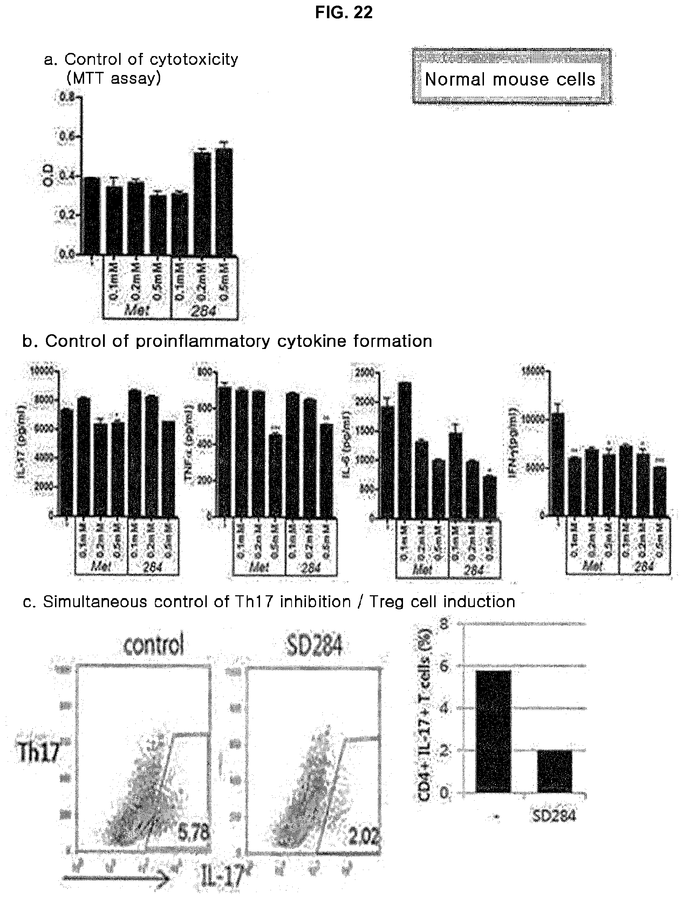

FIG. 22 shows the results obtained by analyzing cytotoxicity, formation of proinflammatory cytokines, and Th17 inhibition & Treg activity facilitation, according to treating the splenocytes derived from normal mouse with the compound SD-284 in predetermined concentrations.

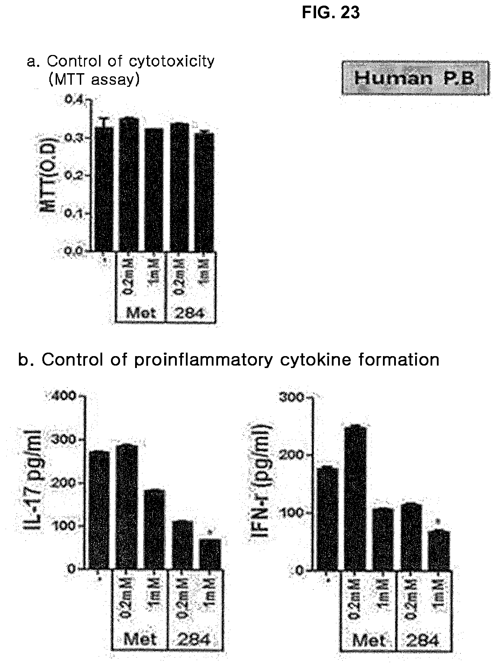

FIG. 23 shows the results obtained by analyzing cytotoxicity and formation of proinflammatory cytokines, according to treating the lymphocytes derived from human peripheral blood with the compound SD-284 in predetermined concentrations.
FIG. 24 shows the results obtained by analyzing inhibitory activities of the novel compounds of the present invention against formation of the proinflammatory cytokines, i.e., TNF-α and IL-17.

### BEST MODE FOR CARRYING OUT THE INVENTION

Described are compounds which can be used as a novel therapeutic agent for effectively preventing or treating immune diseases.

Therefore, the present disclosure may provide a novel compound represented by the following formula or a pharmaceutically acceptable salt thereof: 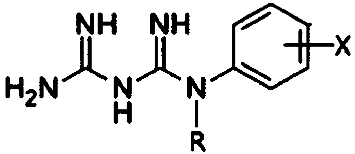 wherein, X is one or more F, Cl, Br or H; and R is hydrogen or an alkyl group.

Preferably, the compound represented by the above formula may be any one of compounds selected from the 24 compounds shown in the following table.

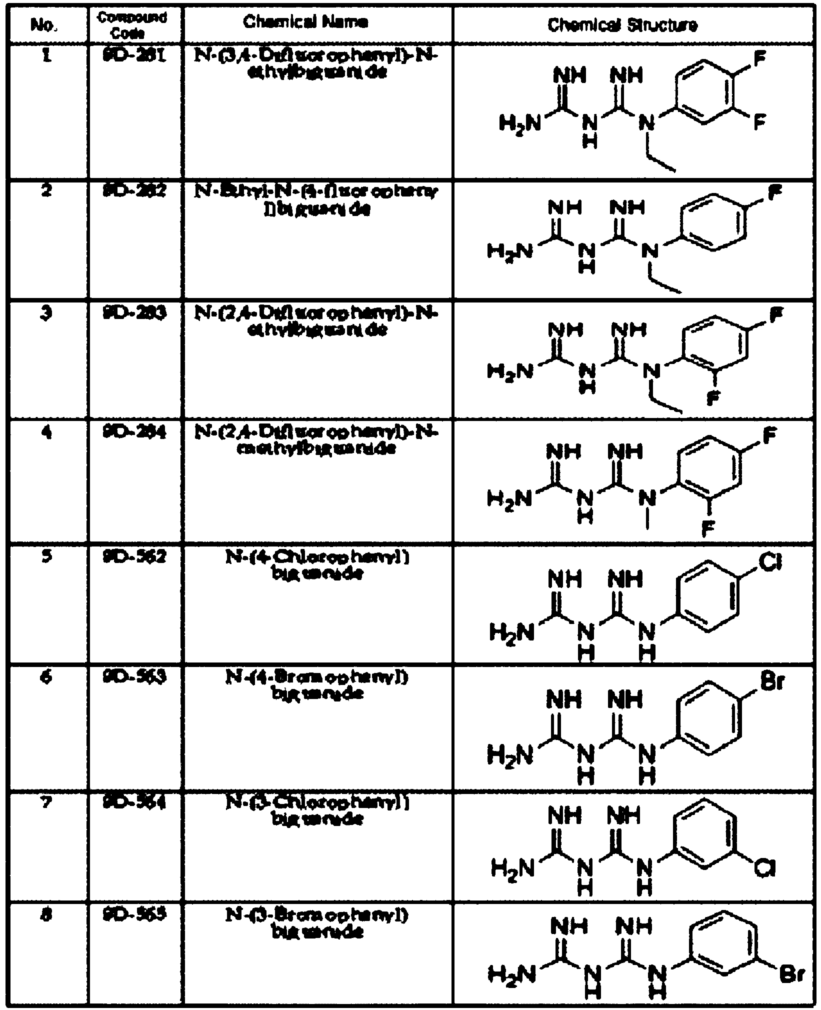

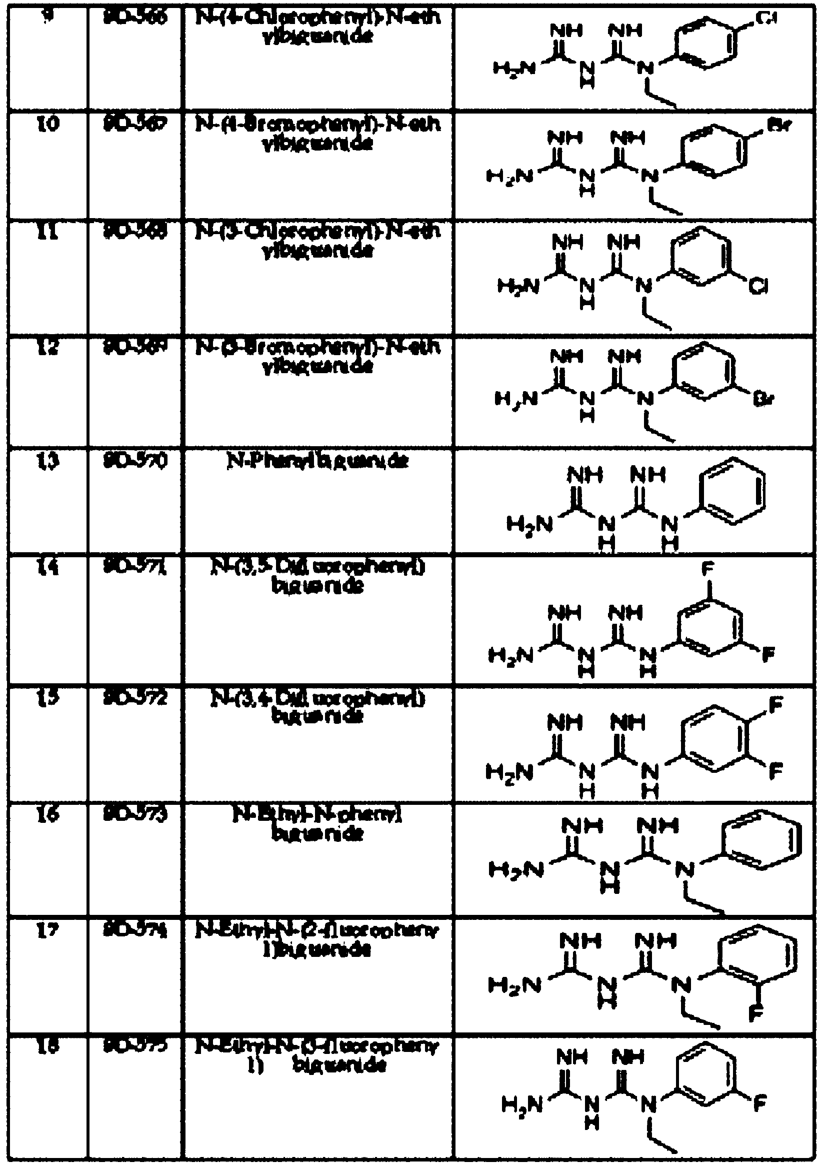

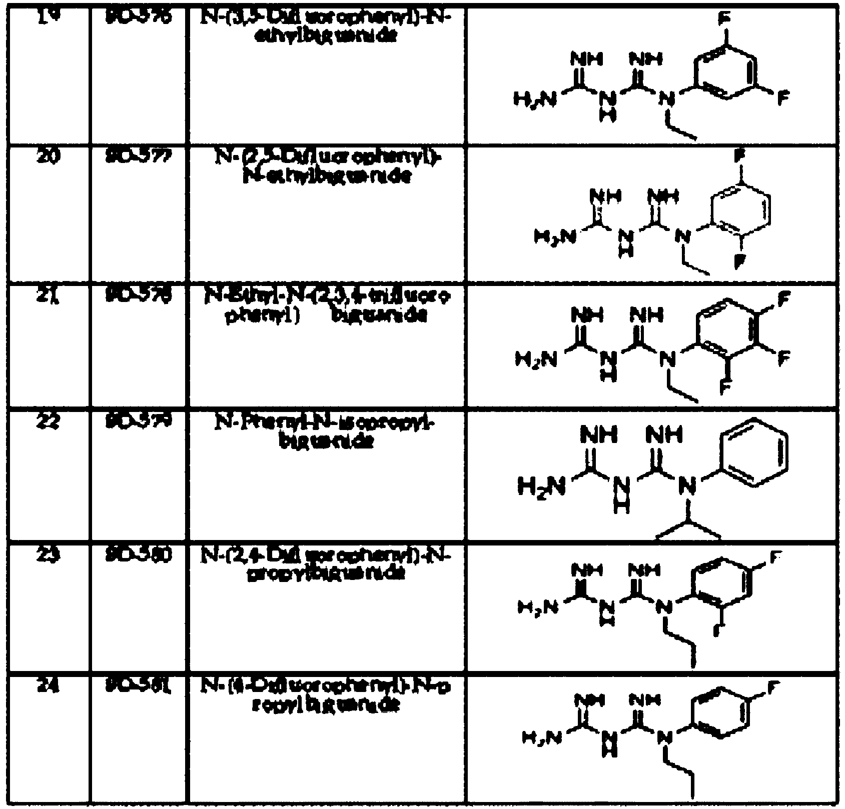

The present inventors carried out the experiments to verify that the compounds synthesized can treat the immune diseases. According to an embodiment of the present disclosure, all the compounds reduce or inhibit proinflammatory cytokine formation, inhibit autoantibody production, inhibit differentiation to osteoclasts. The proinflammatory cytokine may be IL-17, IL-6, TNF-α, IFN-γ, MMP-9 or STAT-3, but not limited thereto.

It has been found that the compounds inhibit production of the autoantibodies, i.e., IgG, IgG1, and IgG2a, thereby showing the modulating activity for inhibiting excessive immune responses.

And also, it has been found that the compounds of the present disclosure have the activities for facilitating or increasing the activity of regulatory T cells and for reducing or inhibiting the activity of pathogenic Th17 cells.

Therefore, it has been confirmed through such results that the synthesized compounds can be used as a novel therapeutic agent for effectively treating immune diseases.

Meanwhile, T cells are one of the cell populations responsible for the central role in the immune system as a biological defense system against various pathogens. T cells are produced in the thymus of the body and then differentiated into the cells having specific properties. The differentiated T cells are classified into Type 1 helper (Th1) cells and Type 2 helper (Th2) cells, in accordance with their functions. The Th1 cells are involved in the cell-mediated immunity and the Th2 cells are involved in the humoral immunity. These two cell populations maintain the balance of the immune system through restraining over-activation thereof each other.

Therefore, most of the immune diseases can be seen to be due to the imbalance between these two immune cells. For example, it is known that abnormal increase in the activity of Th1 cells brings about autoimmune diseases, while abnormal increase in the activity of Th2 cells brings about hypersensitivity-associated immune diseases.

On the other hand, according to the recent studies on the differentiation of Th1 cells, it has been found that there are existed a new population, i.e., regulatory T cells (Treg) modulating the activity of Th1 cells; and thus, there are emerging researches on the treatment of immune diseases using the same. Since the Treg cells have a characteristic to suppress the function of abnormally activated immune cells to control inflammatory responses, various researches for treating immune diseases through increasing the activity of Treg cells are being reported.

In addition to the Treg cells, Th17 cells are formed during the differentiation as another group. Th17 cells are known to be formed during the differentiation of native T cells, according to similar differentiation processes to those of Treg cells. That is, the differentiations to Treg cells and Th17 cells are commonly made in the presence of TGF-β. However, the differentiation to Treg cells does not require IL-6, while the differentiation to Th17 cells is made in the presences of both TGF-β and IL-6. The differentiated Th17 cells are characterized by the secretion of IL-17.

It has been found that Th17 cells, different from Treg cells, are involved in the first line of the inflammatory response shown in immune diseases to maximize the signals of the inflammatory response, thereby accelerating the progression of the disease. Therefore, in case of the autoimmune diseases that are not controlled by Treg cells, developments of a therapeutic agent of autoimmune diseases for targeting the inhibition of Th17 cell activity have been significantly highlighted.

However, although immunosuppressive agents for blocking the T cell signaling pathways are the most widely used as a therapeutic agent of immune diseases currently in use, such immunosuppressive agents have side effect problems, e.g., toxicity, infection, lymphoma, diabetes, tremor, headache, diarrhea, high blood pressure, nausea, renal failure, etc.

In addition to the method for treating immune diseases through inhibiting the activation of T cells, the therapy for controlling the amount of cytokines secreted from immune cells and the therapy using an antibody to target the cytokines secreted from immune cells are under development. However, the former method takes a long time to be applied to patients through the clinical trials, and the method using an antibody has a problem to require high cost in the antibody production processes.

In these respects, the compounds provided by the disclosure have the functions that the inhibition of proinflammatory cytokine formation, the inhibition of Th17 cells, and the activation of Treg cells can be simultaneously operated, which is expected to be able to treat immune diseases more effectively than the conventional therapeutic agents.

Furthermore, according to the results of an embodiment of the present disclosure, it has been confirmed that the compounds of the present disclosure also have an inhibitory activity against the STAT3 gene expression. Recently, activated forms of STAT1, STAT3 and STAT5 are found in various carcinomas. The STAT3 among them is activated in various solid tumors such as breast cancer, head and neck cancer, melanoma, ovarian cancer, lung cancer, pancreatic cancer, prostate cancer as well as blood cancer such as leukemia; and therefore has been used as a target for anticancer agents (Hua Yu and Richard Jove, Nature Review Cancer., 2004, 8, 945).

In addition, it has been known that the activation of STAT3 inhibits apoptosis, induces angiogenesis, and induces immune privilege (Wang T. et al., Nature Medicine., 2004, 10, 48). Therefore, from the facts that inhibition of the STAT3 activation can control tumors through complex anti-cancer mechanisms and that the STAT3 protein involves in various cell functions as well as tumors, the inhibitor thereof may be developed as an immunosuppressive agent.

In addition, there are some cases that the immune system under normal conditions not only controls specific immune responses to autoantigens but also suppresses immune response against foreign antigens. The examples include the response of pregnant women to the fetus; and the immune response to microorganisms in the state of chronic infection. These phenomena are known to be induced by clonal deletion, anergy, and active control by the immunoregulatory T cells (Treg) as a mechanism inducing antigen-specific immune tolerance. The investigations on some patients who accidentally acquired immune tolerance; and the animal models which immune tolerance are experimentally induced have revealed that all the three mechanisms involve in immune tolerance in transplantation. Recently, immunoregulatory T lymphocytes attract attention as a critical cell involved in controlling almost all the immune responses in living body, e.g., autoimmune response, immune response to tumor, immune response to infection, and the like, as well as immune response in transplantation.

Immunoregulatory T cells [i.e., immunomodulatory T lymphocytes (Treg)], which were found recently, can be classified into a natural Treg cell and an adaptive Treg cell. The natural Treg cells, i.e., CD4+ CD25+ T cells, have immunosuppressive function from the time when the cells are newly formed in the thymus; and exist in the frequency of 5 to 10% in the peripheral CD4+ T lymphocytes of normal individuals. Although immunosuppressive mechanism of the cells have not been specifically identified so far, it is recently found that the transcriptional factor Foxp3 plays an important role in the differentiation and activity thereof. In addition, when peripheral natural T cells are stimulated by autoantigens or foreign antigens under specific environments, the cells may be differentiated into the cells showing the immunosuppressive effects, which is called as adaptive or inducible Treg cells. The IL-10-secreting Tr1 cells and the TGF-β-secreting Th3 and CD8 Ts cells, or the like are included therein.

T cells are also differentiated to Th17 cells through differentiation process, in addition to Treg cells. The differentiation to Th17 cells is made in the presence of TGF-β, as in the differentiation to Treg cells. However, although the differentiation to Treg cells does not require IL-6, the differentiation to Th17 cells is made in the presences of both IL-6 and TGF-β. The differentiated Th17 cells are characterized by the secretion of IL-17.

Furthermore, Th17 cells have a characteristic to maximize the signals of the inflammatory response, thereby showing cytotoxicity which accelerates the progression of the disease. Therefore, the inhibition of differentiation to Th17 cells or the inhibition of the activity thereof is one of the methods for treating immune diseases.

In addition, Treg cells express Foxp3. Foxp3, which mainly exists in the regulatory T cells derived from the thymus, is a transcriptional factor residing in the cells having CD4+ CD25+ surface antigens. At the time when the Foxp3-expressing T cells recognize antigens, the Foxp3 makes them have low activity against the antigen. And also, the Foxp3 has a role as a suppressor T cell to inhibit IL-2 formation and cellular division against the potentially autoimmunity-inducible T cells among the thymus-derived differentiated CD4+ CD25- T cells that do not express Foxp3. In addition, it has been found that the Foxp3 has the functions to inhibit not only the transcriptional control of IL-2 but also the transcriptional control of IL-4, IFN-gamma, etc. Therefore, the Foxp3-expressing T cells acting as in the above have been applied in the treatment of immune diseases through their activity of suppressing or regulating the immune response. In addition, there have been attempts to apply as a cell therapy, through increasing the number of human Foxp3-expressing CD4 T cells by treating their self-antigen specific T cell clones with the high concentration of IL-2 cytokine and co-treating with anti-CD3 and anti-CD28 antibodies.

Therefore, from the state of technical developments relating to conventional methods for treating immune diseases, the compounds of the present disclosure show more effective pharmacological effects, along with providing the solution to the problems that were not solved in the conventional art; and therefore may be used very usefully as a therapeutic agent for the treatment of immune diseases.

Accordingly, the pharmaceutical composition for preventing or treating immune diseases of the present disclosure may comprise the novel compounds or their pharmaceutically acceptable salts. Preferably, the salts may be an acid addition salt derived from a pharmaceutically acceptable free acid. As the free acid, an organic acid and an inorganic acid may be used. The organic acid includes citric acid, acetic acid, lactic acid, tartaric acid, maleic acid, fumaric acid, formic acid, propionic acid, oxalic acid, trifluoroacetic acid, benzoic acid, gluconic acid, metasulfonic acid, glycolic acid, succinic acid, 4-toluenesulfonic acid, glutamic acid and aspartic acid, but not limited thereto. The inorganic acid includes hydrochloric acid, hydrobromic acid, sulfuric acid and phosphoric acid, but not limited thereto.

The compounds according to the present disclosure may be isolated from natural products or prepared by using chemical synthetic methods known in the art. The present inventors prepared the compounds through the syntheses according to the methods described in the following Examples.

As used herein, the term "immune disease" refers to the diseases that components of the mammalian immune system cause, mediate, or contribute to pathological conditions in mammals. And also, said term may include all of the diseases that stimulation or interruption of the immune response leads to compensatory effects on the progression thereof; and may include the diseases caused by hypersensitive immune response. Examples of the immune disease include autoimmune diseases; inflammatory diseases; and transplantation rejections of cells, tissues or organs, but not limited thereto.

And also, as one of the most important characteristics, all normal individuals has an ability to recognize, response to and remove non-self-antigens, while they do not harmfully response to the self-antigenic substances. The non-response is referred to as 'immunologic unresponsiveness' or 'tolerance'.

However, any problems in inducing or maintaining self-tolerance result in immune response to the self-antigens, thereby leading to a phenomenon of attacking one's own tissues. The disease caused by these processes is referred to as "autoimmune disease".

In addition, the term "inflammatory disease" refers to the diseases caused by inflammatory substances (proinflammatory cytokines) such as TNF-α (tumor necrosis factor-α), IL-1 (interleukin-1), IL-6, prostaglandin, leukotriene or nitric oxide (NO), which are secreted from immune cells, e.g., macrophage, according to excessive enhancement of the immune system due to harmful stimuli such as inflammation-inducing factors or irradiation.

And also, in order to ensure successful organ transplantation, it is required to overcome immune rejection between the transplanted cells and the recipient's organ. T cells are the main mediators of transplant immune rejection. The T cell receptors recognize the major histocompatibility complexes (MHC) expressed on the graft to induce immune response, which leads to transplant rejection. The major histocompatibility complexes depend on the type of glycoprotein antigens thereof. Because the immune responses due to mismatch of the histocompatibility antigens are an obstacle hindering successful transplantation, the accuracy of histocompatibility antigen examination and the investigation on match of the histocompatibility antigens are critical elements.

Human has various histocompatibility antigens, e.g., Class I antigens such as HLA-A, -B, and -C; and Class II antigens such as HLA-DR, -DP, and -DQ. The biological function of these antigens is to deliver an antigen to T cells. The Class I antigens are expressed in most of nucleated cells; and the antigens delivered thereby are recognized by CD8+ cytotoxic T lymphocytes. The Class II antigens are expressed in dendritic cells known as antigen-presenting cells, B lymphocytes, activated T lymphocytes, macrophages, etc.; and deliver antigens to CD4+ T lymphocytes. The T lymphocytes recognize the antigens through binding the antigens delivered to T cells to the receptors on the T lymphocytes. In the course of the transplantation, the T lymphocytes recognize the histocompatibility antigens derived from the other person in higher frequency than one's own histocompatibility antigens. 1% to 10% of all T lymphocytes in a donors or a patient recognizes the histocompatibility antigens derived from the patient or the donor; and proliferate in response thereto, thereby causing a series of immune responses, which is called as "alloresponse". And also, the immune response that donor's T lymphocytes cause against patient's histocompatibility antigens is called as "graft-versus-host disease (GVDH)". In contrast, the immune response that patient's T lymphocytes cause against donor's histocompatibility antigens is called as "graft rejection".

Therefore, immunosuppressive agents are used to reduce abnormal responses due to immune responses occurred during the transplantation. The immunosuppressive agents commonly suppress T cell-mediated immune responses to the grafts. Recently, the methods for treating transplant rejection diseases through suppressing immune responses using regulatory T cells have been being attempted.

In addition, the immune diseases that can be prevented and treated in the present invention may include rheumatoid arthritis, Behcet's disease, polymyositis or dermatomyositis, autoimmune cytopenia, autoimmune myocarditis, atopic dermatitis, asthma, primary cirrhosis, dermatomyositis, Goodpasture's syndrome, autoimmune meningitis, Sjogren's syndrome, lupus, Addison's disease, alopecia areata, ankylosing myelitis, autoimmune hepatitis, autoimmune mumps, Crohn's disease, insulin-dependent diabetes, dystrophic epidermolysis bullosa, epididymitis, glomerulonephritis, Graves' disease, Guillain-Barre syndrome, Hashimoto's disease, hemolytic anemia, multiple sclerosis, myasthenia gravis, pemphigus vulgaris, psoriasis, rheumatic fever, sarcoidosis, scleroderma, spondyloarthropathy, thyroiditis, vasculitis, vitiligo, myxedema, pernicious anemia, mitochondria-related syndrome and ulcerative colitis, but not limited thereto.

Therefore, the composition according to the disclosure can be used as a pharmaceutical composition for preventing or treating immune diseases.

The term "therapy" means, unless otherwise stated, reversing or alleviating the diseases or disorders that said term is applied or at least one symptom of the disease or disorders; or inhibiting or preventing the progress thereof. The term "treating" as used herein refers to the treating act when the "therapy" is defined as above. Therefore, "treating" or "therapy" of immune diseases in the mammal may include one or more of the followings:
(1) inhibiting the growth of immune diseases, i.e., blocking the development thereof,
(2) preventing the spread of immune diseases, i.e., preventing the metastasis thereof,
(3) alleviating immune diseases,
(4) preventing the recurrence of immune diseases, and
(5) palliating the symptoms of immune diseases.

The composition for preventing or treating immune diseases according to the present disclosure may comprise a pharmacologically effective amount of one or more of the compounds of the present of the present disclosure or their salts alone, or a pharmacologically effective amount of one or more of the compounds of the present disclosure or their salts along with one or more pharmaceutically acceptable carriers, additives or diluents. The pharmacologically effective amount refers to the amount sufficient to prevent, improve and treat the symptoms of immune diseases.

The pharmacologically effective amount of the compounds of the present disclosure or their salts may be appropriately changed according to severity of immune disease symptoms, the patient's age, body weight, health, sex, administration route and treatment period, etc.

The expression "pharmaceutically acceptable" refers to a physiologically acceptable composition which does not cause gastrointestinal disorders, allergic reactions or similar reactions thereto at the time when the composition is administered to a human being. Examples of the carriers, additives, and diluents include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate and mineral oil. In addition, fillers, anti-coagulants, lubricants, wetting agents, flavoring agents, emulsifying agents and preservatives may be further included.

And also, the composition of the present disclosure may be formulated using methods known in the art so as to provide rapid, sustained or delayed release of the active ingredient after being administered to the mammal. The formulations may be in
the form of powders, granules, tablets, emulsion, syrup, aerosol, soft or hard gelatin capsules, sterile injectable solutions, or sterile powders.

And also, the composition for preventing or treating immune diseases according to the invention may be administered via various routes, including orally, transdermally, subcutaneously, intravenously, intramuscularly. The dose of the active ingredient may be appropriately selected according to various factors such as administration route, patient's age, sex, body weight, and severity. The composition for preventing or treating immune diseases according to the invention may be administered in combination with other known compounds having an effect of the prevention, improvement or treatment of symptoms of immune diseases.

The present disclosure also provides a use of the composition comprising the compounds as an active ingredient for the manufacture of a medicament for preventing or treating immune diseases. The composition of the present disclosure comprising the compounds of the present disclosure as an active ingredient can be used as a use for the manufacture of a medicament for preventing or treating immune diseases.

As used herein, the term "mammal" refers to a mammal that is the subject of treatment, observation or experiment, and preferably refers to a human.

The expression "therapeutically effective amount" as used herein means the amount of the active ingredient or the pharmaceutical composition for inducing a biological or medical response in the tissue system, animal, or human, which is determined by a researcher, a veterinarian, or a physician or other clinician. The expression includes the amounts that result in relief of the symptoms of the disease or disorder being treated. The therapeutically effective dose and administration frequency of the active ingredient of the present invention will be varied according to the desired effects; and obvious to those skilled in the art. Therefore, the optimum dose to be administered may be readily determined by those skilled in the art; and adjusted depending on various factors, including type of the disease, severity of the disease, amount of the active ingredient and other components contained in the composition, type of formulation, and patient's age, body weight, general health status, sex, diet, administration time, administration route and secretion ratio of the composition, treatment period, and coadministered drugs.

In addition, the present disclosure may provide an immunomodulator comprising the compound of the present disclosure as an active ingredient.

And also, the present disclosure may provide a method for reducing the activity of Th17 cells, which comprises treating cells with the compounds of the present invention *in vitro;* and a method for increasing the activity of Treg cells, which comprises treating cells with compounds of the present invention *in vitro.*

### MODE FOR CARRYING OUT THE INVENTION

The invention present will be described in more detail in reference to the following examples. These examples are intended to illustrate the present invention more specifically, but the scope of the present invention is not intended to be limited to these examples.

The compounds *per se* or compositions comprising them do not form part of the invention.

### <Example 1>

### Synthesis of compounds of the disclosure having the effect for treating immune diseases

The compounds represented by the formulas shown in Table 1 were prepared in the following reaction scheme. Specifically, after each aniline compound (1.0 mmol) was dissolved in acetonitrile solvent in a sealable reactor, dicyanodiamide (1.0 mmol) and concentrated sulfuric acid (1.0 mmol) were added thereto. After sealing the reactor, the reaction mixture was stirred at 175 °C for 1 hour. The reaction mixture was cooled to room temperature to produce the white solid. After removing the solvent, the resulting sold was washed with hexane and isopropyl alcohol to synthesize the compounds as a white solid.

All the 24 compounds were synthesized in the same conditions, except for using the different aniline compounds. The aniline compounds used in the syntheses of the respective compounds and the chemical names and chemical structures of the synthesized compounds are described in the following table.

The representative reaction scheme used for synthesizing the compounds is shown below.

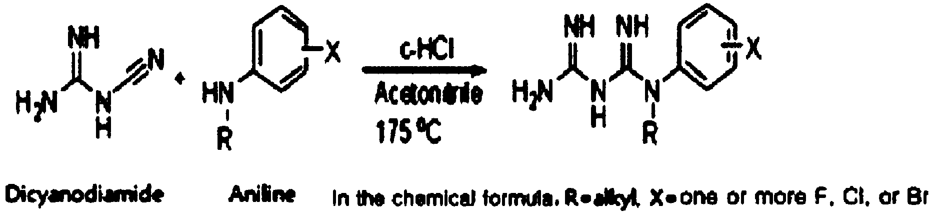

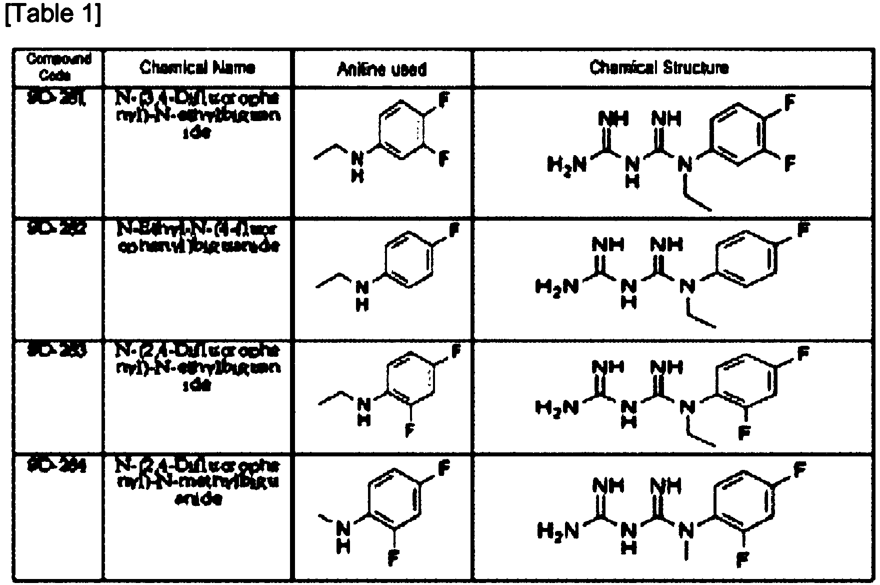

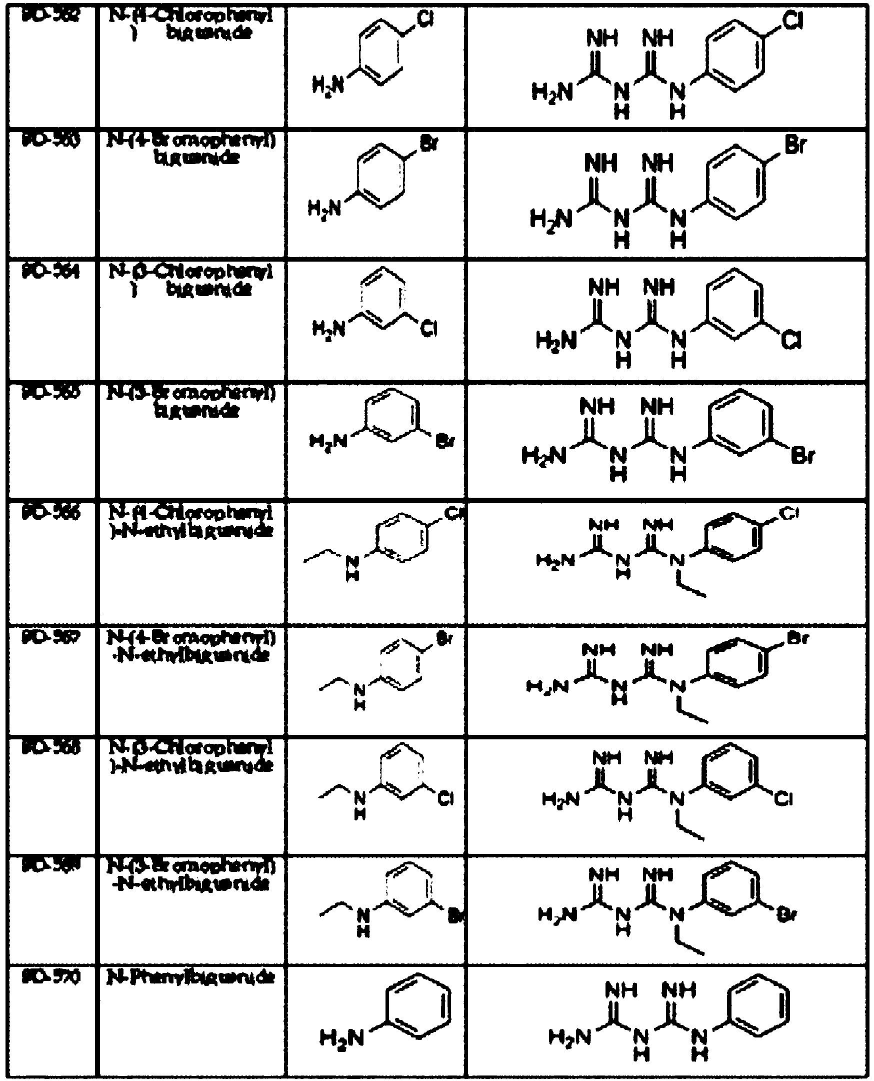

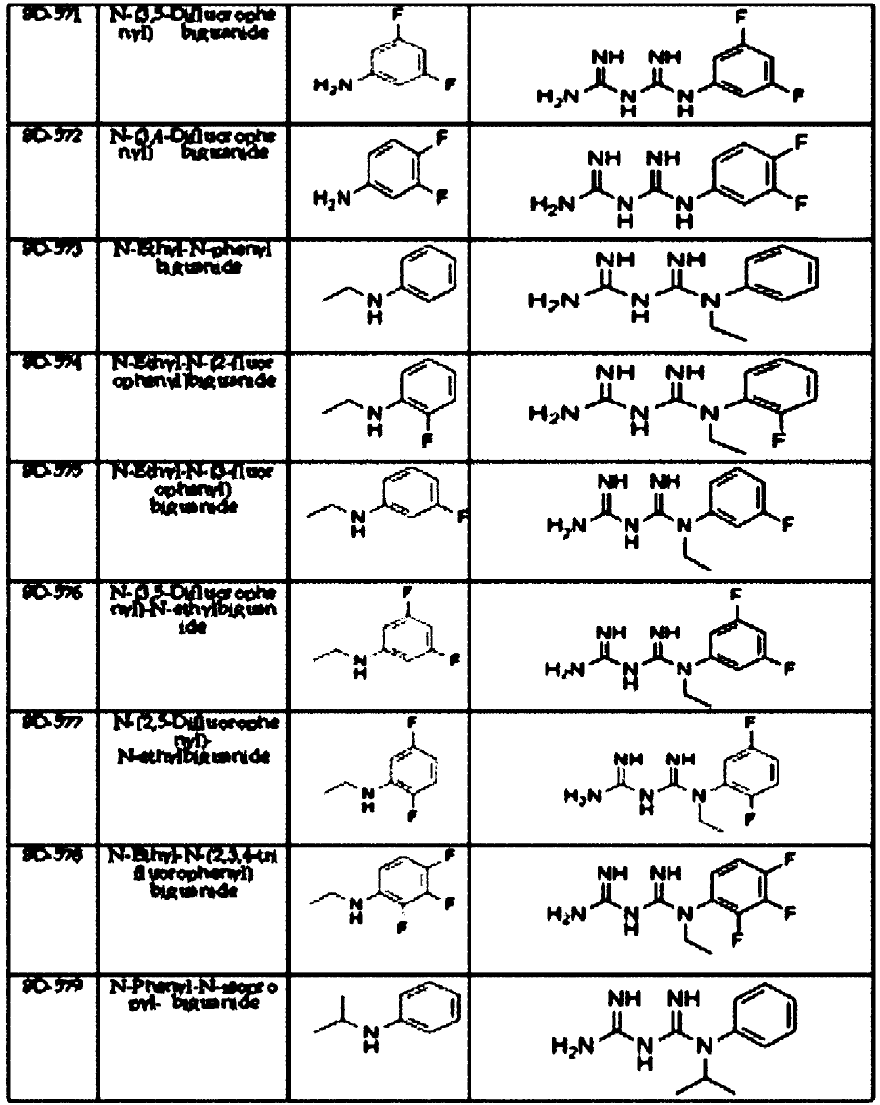

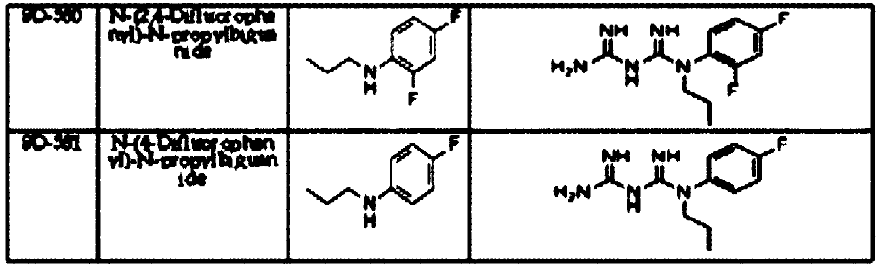

The respective derivatives of the present invention synthesized as in the above were identified by ¹H-NMR analysis and the results thereof are shown in the following table.

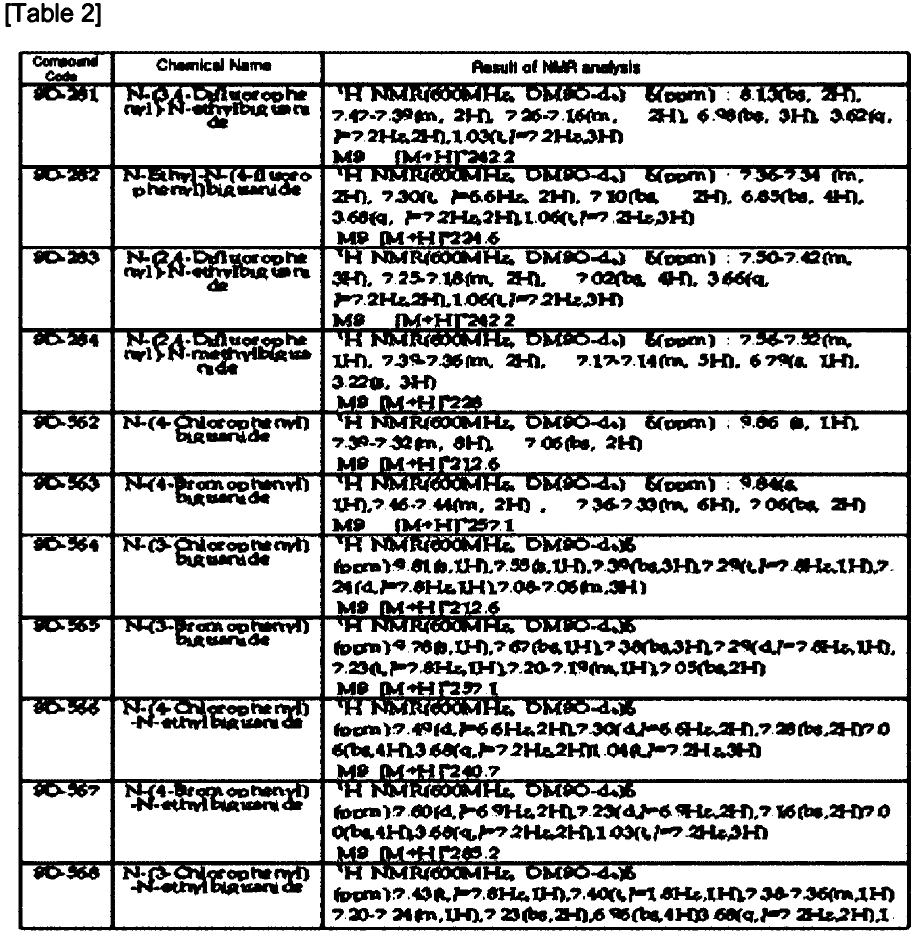

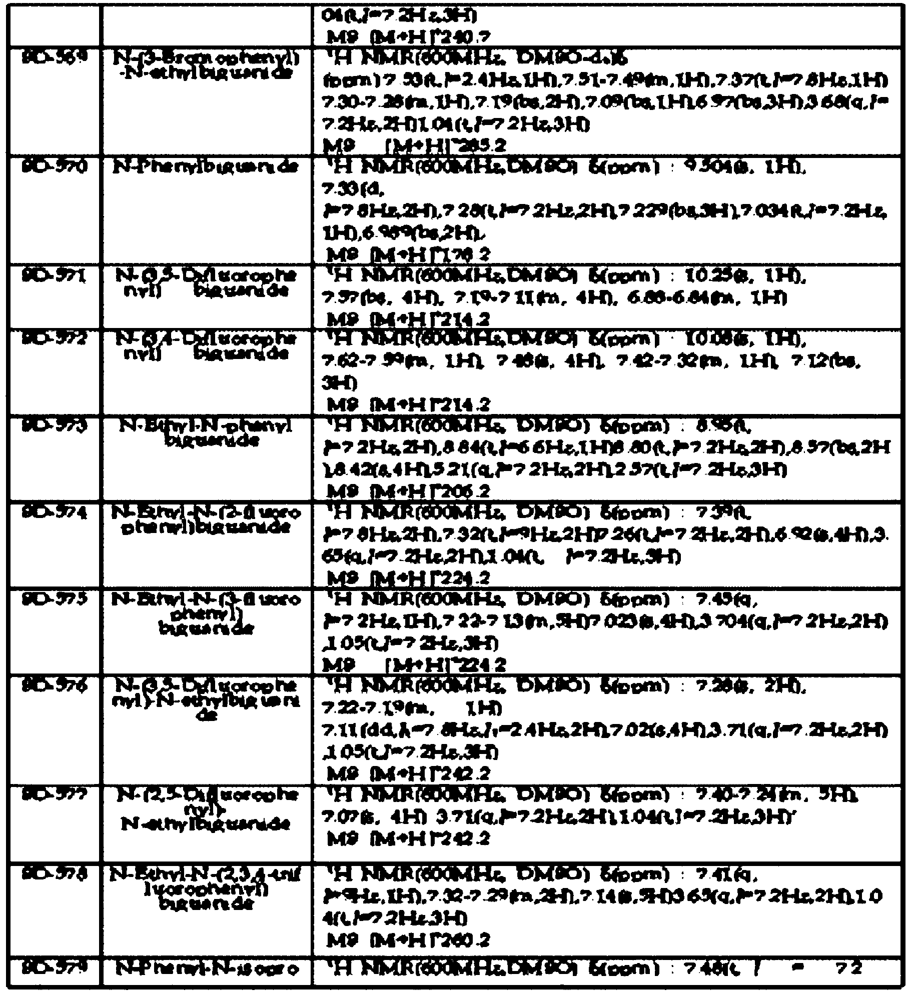

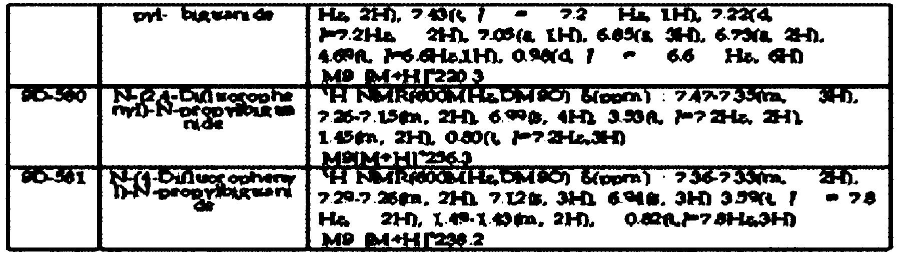

### <Experimental Method>

### <1> Cells analyzed

The present inventors carried out the following experiments based on the following experimental groups, in order to confirm that the respective compounds synthesized in the present disclosure can treat and prevent immune diseases.

**[Table 3]**

| Experimental groups carried out in the experiments of the present invention | |
|---|---|
| Experimental group | Cells for experiments |
| Normal mouse group | Splenocytes and BM cells derived from normal mouse (DBA/1J mouse) |
| Rheumatoid arthritis-induced mouse group | Splenocytes and BM cells derived from rheumatoid arthritis-induced DBA/1J mouse by CIA treatment |
| Lupus-induced mouse group | Splenocytes and BM cells derived from lupus-induced sanroque mouse |
| Human P. B group | lymphocytes derived from human peripheral blood (P.B) |

Specifically, in the normal mouse group, the splenocytes derived from normal DBA/1J mouse were activated by culturing under the stimulating condition of anti-CD3 antibody (0.5 µg/ml) for 72 hours, before treatment of the compounds of the present invention.

And also, the rheumatoid arthritis-induced mice were prepared using DBA/1J normal mice. The solution of Type II collagen (CII) in 0.1N acetic acid solution (4 mg/ml) was dialyzed in the dialysis buffer (50 mM Tris, 0.2N NaCl) and then mixed with the same volume of the complete Freund's adjuvant (CFA, Chondrex) containing M. tuberculosis. The resulting immunogen was subcutaneously injected into the tail base of the normal mice, in 100 µl per mouse (i.e., 100 µl/100 µg) (primary injection). After 2 weeks therefrom, the solution (100 µl) obtained by mixing the CII with the same volume of the incomplete Freud's adjuvant (IFA, Chondrex) was injected into one of hind legs (foot pad) (i.e., 100 µl/100 µg) (secondary injection) to prepare the rheumatoid arthritis-induced mice. The splenocytes derived from the prepared rheumatoid arthritis-induced mouse were activated by culturing under the stimulating condition of anti-CD3 antibody (0.5 µg/ml) for 72 hours, and then used in the experiments.

In addition, as the lupus-induced mouse model group, we used the sanroque mice used as a lupus model in the art. In the human P. B group, we used the lymphocytes derived from human peripheral blood. The isolations of splenocytes and lymphocytes from the mice and human peripheral blood were performed according to conventional methods known in the art. All the isolated cells were activated by culturing under the stimulating condition of anti-CD3 antibody (0.5 µg/ml) for 72 hours, and then used in the experiments.

### <2> Cytotoxicity assay (MTT assay)

The MTT assay was carried out to determine whether the compounds synthesized in the present invention cause cytotoxicity. The respective cells were added to each well of a 96 well plate (2 × 10⁵ cells per well), treated with the compounds of the present invention in predetermined concentrations, and then incubated for 72 hours. The MTT solution (0.5%, 3-(4,5-dimethylthiazol-2-yl)-2,5-diphenyl-2H-tetrazolium bromide) was added to the respective cells, which were then incubated additionally for 4 hours. The absorbance at 540 nm was measured with an ELISA (enzyme-linked immunospecific assay) reader to determine the cytotoxicity.

### <3> Analysis of the control against the immune response by autoantibody production

The enzyme-linked immunospecific assay (ELISA) was performed to determine whether the novel compounds of the present invention affect the productions of total IgG, total IgG1 and IgG2a antibodies in the serum. That is, the total IgG level and the antibody-specific IgG1 and IgG2a levels were measured in the cells treated with the compounds of the present invention, using the sandwich ELISA. In a 96-well plate, each monoclonal anti-mouse IgG and the CII were reacted for 1 hour at room temperature. The blocking solution (1% BSA/PBST) was added thereto to block non-specific bindings. The mouse control serum was subject to sequential half dilutions and then used as a standard. The cell culture supernatant was added to the reaction mixture, which was then reacted for 1 hour at room temperature. And then, the anti-mouse IgG-HRP and anti-mouse IgG2a-HRP were reacted for 1 hour at room temperature, followed by washing four times. The reaction mixture was subject to color development with TMB system and then the absorbance at 450 nm wavelength was measured.

### <4> Analysis of the effects on the proinflammatory cytokine formation

The formation levels of IL-17, IL-6, TNF-α, IFN-γ, MMP-9, and STAT-3 and the mRNA expression levels thereof were measured to determine whether the compounds of the present disclosure can affect formation of the proinflammatory cytokines, which are causative materials of inflammation and immune diseases. The supernatant was obtained from the cultured cells that had been treated with the compounds of the present invention; and then analyzed using the enzyme-linked immunospecific assay (ELISA) method to determine the formation levels of the proinflammatory cytokines. Specifically, the respective cells were reacted with the cytokine-specific antibodies (anti-IL-17, anti-IL-6, anti-TNF-α, anti-IFN-γ, anti-MMP-9, and anti-STAT-3 antibodies) at 4 °C overnight. The blocking solution (1% BSA/PBST) was added thereto to block non-specific bindings. And then, the respective biotinylated antibodies were reacted for 2 hours at room temperature, followed by washing 4 times. The diluted ExtraAvidin-Alkaline Phosphatase conjugate was added thereto and then reacted at room temperature for 2 hours. The reaction mixture was subject to color development with the PNPP/DEA solution and then the absorbance at 405 nm wavelength was measured.

In addition, in order to analyze the amounts mRNA, the total RNAs were obtained from the cells. And then, the RT-PCR was carried out using the primers specifically bound to IL-17, IL-6, TNF-α, and IFN-γ, to determine the expression levels of proinflammatory cytokines.

### <5> Analysis of the activities for inhibiting Th17 cells and inducing Trea cells

Furthermore, the present inventors performed the flow cytometry assay to determine whether the compounds of the disclosure not only inhibit differentiation and activity of the Th17 cells associated with inflammation but also facilitate activity of the Treg cells having immunomodulatory function. That is, T cells were cultured under the condition for differentiation to Th 17 cells or under the condition for differentiation to Treg cells; and then the number of Foxp3+ Treg cells or the number of IL-17+ Th 17 cells was measured with a flow cytometry instrument.

### <6> Inhibitory effect against the differentiation to osteoclasts

In order to investigate the effect of the compounds of the present disclosure on the differentiation to osteoclasts, the obtained mouse bone marrow cells were induced to differentiate in the presences of MCSF (macrophage colony-stimulating factor) and soluble RANKL (Sugatani et al. 2003, J. Cell. Biochem. 90, 59-67). The bone marrow cells were prepared from the femur and tibia of 6-week-old mice and then maintained, in the presence of M-CSF (30 ng/ml: R&D Systems, Minneapolis, MN), at 37°C in an eight-well chamber slide (3 × 10⁵ cells/well; Nalge Nunc International, Naperville, IL). After 3 days therefrom, non-adhesive cells including lymphocytes were removed and then the adhesive osteoclast progenitor cells were cultured for another four days in the presences of M-CSF (30 ng/ml) and RANKL (30 ng/ml; Strathmann, Hamburg, Germany) to obtain the osteoclasts. The cell culture medium was exchanged once during the presences of M-CSF and RANKL. The cells were fixed and then the TRAP (tartrate-resistant acid phosphatase) staining was performed with the staining kit (Sigma) according to the manufacturer's protocol. Each chamber was observed with a microscope (40X magnification) and the TRAP-positive multinucleated cells containing more than three nuclei were counted as osteoclasts (Sugatani. et al. 2003, J. Cell. Biochem. 90, 59-67).

And also, the bone marrow cells were differentiated to osteoclasts and treated with the compounds of the present disclosure in the predetermined concentrations in the presences of M-CSF and RANKL. After culturing for 48 hours, the cells were subject to the TRAP staining and then the TRAP-positive multinucleated cells were counted.

### <Experimental Example 1 >

### Cytotoxicity assay results of the compounds of the present disclosure

In order to determine whether the compounds synthesized in Example 1 cause cytotoxicity, cell viability of each group of cells described in Table 3 was measured through the MTT assay. That is, the cells of each group (2 × 10⁵ cells) were treated with the compounds synthesized in the present disclosure and then the cell viabilities thereof were analyzed.

As the results thereof, the compounds SD-281, SD-282, SD-283, and SD-284 did not show cytotoxicity according to the treatments thereof, when compared with the control group or the metformin-treated group. Therefore, it has been confirmed that these compounds have no cytotoxicity in both normal cells and disease-induced cells.

### <Experimental Example 2>

### Analysis results of the control of the compounds of the present disclosure against the immune response by autoantibody production

In order to investigate effects of the compounds synthesized in Example 1 on the immune response by autoantibody production, the blood samples were collected from the orbital sinus of each mouse in the four groups described above and then the levels of IgG, IgG1 and IgG2a in each serum were measured.

As the results thereof, the amounts of the immunoglobulins (total IgG, IgG1, and IgG2), which reflect autoantibody production status in disease conditions, were reduced according to the treated concentrations of all the compounds of the present disclosure. It has been confirmed that the compounds described in Table 4 have an excellent immunemodulating activity for suppressing excessive immune responses.

**[Table 4]**

| | Total IgG | | | IgG1 | | | IgG2 | | |
|---|---|---|---|---|---|---|---|---|---|
| | A | B | C | A | B | C | A | B | C |
| Control | 11 | 83.2 | 141.5 | 311.4 | 286 | 422.3 | 103.2 | 109.6 | 151.9 |
| SD-281 | 2.3 | 58.1 | 124.5 | 83.8 | 135.2 | 258.2 | 35.6 | 33.5 | 84.3 |
| SD-282 | 0.7 | 60.3 | 127.7 | 1 | 218.7 | 425.4 | 37.1 | 38.3 | 110.9 |
| SD-283 | 2.9 | 49.6 | 113.5 | 41 | 135.3 | 296.5 | 97.6 | 99.4 | 76 |

| | | | | | | | | | |
|---|---|---|---|---|---|---|---|---|---|
| Unit: ng/ml A: Normal mouse cells, B: Rheumatoid arthritis-induced mouse cells, C: Lupus-induced mouse cells, D: Lymphocytes derived from human peripheral blood | | | | | | | | | |

### <Experimental Example 3>

### Results of inhibition analysis against formation and gene expression of proinflammatory cytokines

In order to determine whether the compounds of the present disclosure inhibit formation of the proinflammatory cytokines inducing inflammation and immune diseases and also inhibit the proinflammatory cytokines in gene level, we carried out the experiments according to analysis of the effects on the proinflammatory cytokine formation described in the above <4>.

As the results thereof, all the compounds synthesized in the present disclosure inhibited formations of the proinflammatory cytokines, i.e., IL-17, IL-6, TNF-α, IFN-γ, MMP-9 and STAT-3 and expressions of the genes thereof in a concentration-dependent manner. Therefore, it can be seen from these results that the compounds of the present disclosure prevent and treat immune diseases through inhibition of proinflammatory cytokine formation.

**[Table 5]**

| Analysis of the effects of the compounds of the present disclosure on the proinflammatory cytokine formation | | | | | | | | | | | | | | | | |
|---|---|---|---|---|---|---|---|---|---|---|---|---|---|---|---|---|
| | IL-17 | | | | IL-6 | | | | TNF-α | | | | IFN-γ | | | |
| | A | B | C | D | A | B | C | D | A | B | C | D | A | B | C | D |
| Control | 3.2 | 3.7 | 0.9 | 0.3 | 2.2 | 1.9 | 1.8 | | 0.9 | 0.1 | 1.3 | | 9.4 | 8.3 | 14.5 | 0.2 |
| SD-281 | 1.7 | 2.2 | 0.6 | 0.1 | 1.4 | 1.4 | 1 | | 0.5 | 0.4 | 0.7 | | 5.8 | 5.3 | 10 | 0.1 |
| SD-282 | 1.7 | 2.1 | 0.6 | 0.1 | 1.3 | 1.3 | 1 | | 0.4 | 0.4 | 0.7 | | 3.3 | 2.9 | 7.6 | 0.1 |
| SD-283 | 1.6 | 2.2 | 0.4 | 0.1 | 1.4 | 1.1 | 0.9 | | 0.5 | 0.3 | 0.6 | | 7.2 | 5.4 | 5.5 | 0.1 |
| SD-284 | | | | 0.1 | | | | | | | | | | | | 0.08 |

| | | | | | | | | | | | | | | | | |
|---|---|---|---|---|---|---|---|---|---|---|---|---|---|---|---|---|
| Unit: ng/ml A: Normal mouse cells, B: Rheumatoid arthritis-induced mouse cells, C: Lupus-induced mouse cells, D: Lymphocytes derived from human peripheral blood | | | | | | | | | | | | | | | | |

**[Table 6]**

| Analysis of the effects of the compound SD-284 on the proinflammatory cytokine formation in normal mice | | | | |
|---|---|---|---|---|
| | IL-17 | IL-6 | TNF-α | IFN-γ |
| Control | 7.6 | 1.8 | 0.7 | 10 |
| SD-284 | 6.5 | 0.7 | 0.5 | 4.9 |

| | | | | |
|---|---|---|---|---|
| Unit: ng/ml | | | | |

**[Table 7]**

| Analysis of the inhibitory effects of the compounds of the present disclosure against the proinflammatory cytokine activity | | | | | | | |
|---|---|---|---|---|---|---|---|
| | TNF-α | | | MMP-9 | | | STAT3 |
| | A | B | C | A | B | C | A |
| Control | 1 | 1 | 1 | 1 | 1 | 1 | 1 |
| SD-281 | 0.2 | 0.6 | 0.1 | 0.5 | 0.4 | 0.4 | 1.2 |
| SD-282 | 0.07 | 0.06 | 0.8 | 0.1 | 0.08 | 1.3 | 0.6 |
| SD-283 | 0.4 | 0.06 | 0.1 | 0.3 | 1 | 0.04 | 1.3 |

| | | | | | | | |
|---|---|---|---|---|---|---|---|
| Unit: gene expression A: Normal mouse cells, B: Rheumatoid arthritis-induced mouse cells, C: Lupus-induced mouse cells | | | | | | | |

### <Experiment 4>

### Results of the analysis of the modulating activities in the Th17 cells and the Trea cells

In order to investigate whether the compounds of the present disclosure control both differentiation and activity of the Th17 cells secreting the proinflammatory cytokine IL-17 and differentiation and activity of the Treg cells having immunomodulatory function, we carried out the analysis of the activities for inhibiting Th17 cells and inducing Treg cells described in the above <5>.

As the results thereof, all the compounds of the present disclosure inhibited both the IL-17 expression in the disease-induced cells and the differentiation to the Th17 cells under the differentiation condition to Th17 cells. And also, all the compounds of the present disclosure increase both the expression of Foxp3 (the marker of immunomodulatory cells) and the number of Foxp3-expressing cells, under the differentiation condition to Treg cells. In addition, it has been found that the compounds can effectively suppress the over-activated Th17 cells.

**[Table 8]**

| Analysis of the effects of the compounds of the present disclosure on the Th17 inhibition and the Treg activity | | | | | | |
|---|---|---|---|---|---|---|
| TCR condition | Th17 | | | Treg | | |
| | A | B | C | A | B | C |
| Control | 2 | 3.4 | 1.1 | 2.6 | 3.3 | 2.8 |
| SD-281 | 1.2 | 1.5 | 0.8 | 2 | 4.6 | 2.8 |
| SD-282 | 1.2 | 1.5 | 0.9 | 2.5 | 4.6 | 2.9 |
| SD-283 | 1.1 | 2.3 | 0.5 | 3 | 5.1 | 3.3 |

| | | | | | | |
|---|---|---|---|---|---|---|
| A: Normal mouse cells, B: Rheumatoid arthritis-induced mouse cells, C: Lupus-induced mouse cells | | | | | | |

**[Table 9]**

| Analysis of the effects of the compounds of the present disclosure on the Th17 cells under the differentiation condition to Th17 cells | |
|---|---|
| Th17 condition | Th17 |
| | A |
| Control | 5.7 |
| SD-281 | 0.4 |
| SD-282 | 0.5 |
| SD-283 | 0.9 |
| SD-284 | 2 |

Therefore, it can be seen from these results that the compounds of the present disclosure can control the Th17 and Treg simultaneously (i.e., not separately) to induce immunomodulatory activity more effectively, thereby being able to be used more efficiently as an immunomodulating agent or a therapeutic agent for immune diseases.

### <Example 5>

### Results of the analysis on inhibition against the differentiation to osteoclasts

In order to investigate whether the compounds of the present disclosure can effectively treat immune diseases, the inhibition levels of the compounds of the present disclosure against the differentiation to osteoclasts in the cells stimulated with M-CSF and RANKL were confirmed through the osteoclast differentiation factor TRAP staining.

As the results thereof, all the compounds of the present disclosure reduced the number of the cells expressing the osteoclast differentiation marker TRAP. It can be seen from these results that the compounds of the present disclosure effectively reduce differentiation potential to the joint destruction-inducing osteoclasts, thereby being able to be used for preventing or treating diseases caused by the differentiation to osteoclasts.

**[Table 10]**

| Analysis of the inhibitory effects of the compounds of the present disclosure against the differentiation to osteoclasts | | |
|---|---|---|
| | TRAP+ cells | |
| | A | B |
| Control | 310 | 246 |
| SD-281 | 196 | 57 |
| SD-282 | 157 | 89 |
| SD-283 | 123 | 85 |

| | | |
|---|---|---|
| A: Normal mouse cells, B: Rheumatoid arthritis-induced mouse cells | | |

### <Example 6>

### Inhibitory effect against formation of the proinflammatory cytokines TNF-α and IL-17 in mouse splenocytes

In addition, in order to evaluate efficacy of the inhibitory effect of the compounds synthesized in Example 1 against proinflammatory cytokines, the cells derived from the spleen of DBA1/J normal mice were treated with an anti-mouse CD3 antibody in the concentration of 0.5 µg/ml and then stimulated with each compound in the concentrations of 200 and 500 uM respectively. The inhibition levels of the proinflammatory cytokines IL-17 and TNFa were determined through the ELISA method.

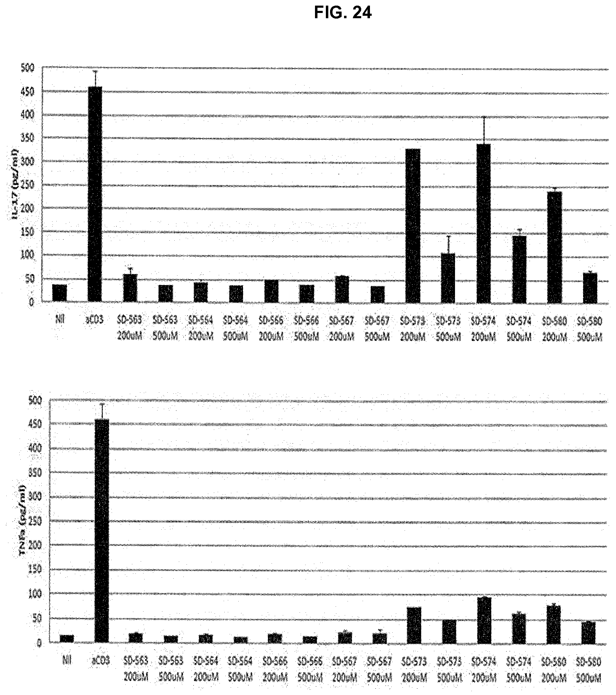

As the results thereof, as shown in FIG 24, it has been found that the compounds SD-563, SD-564, SD-566, SD-567, SD-573, SD-574, and SD-580 have the inhibitory activity against the proinflammatory cytokines IL-17 and TNFα simultaneously and that the inhibitory activities thereof are increased in a concentration-dependent manner.

All changes which come within the meaning and range of equivalency of the claims are to be embraced within the scope of the present invention.

## Claims

1. A pharmaceutical composition for use in facilitating or increasing the activity of regulatory T cells and reducing or inhibiting the activity of pathogenic Th17 cells in an immune disease, comprising N-ethyl-N-(4-fluorophenyl)-biguanide or a pharmaceutically acceptable salt thereof as an active ingredient.

2. The pharmaceutical composition for use of claim 1, wherein the N-ethyl-N-(4-fluorophenyl)-biguanide or a pharmaceutically acceptable salt thereof further reduces or inhibits proinflammatory cytokine formation, inhibits autoantibody production, and inhibits differentiation to osteoclasts.

3. The pharmaceutical composition for use of claim 2, wherein the proinflammatory cytokine is IL-17, IL-6, TNF-α, IFN-γ, MMP-9 or STAT-3.

4. The pharmaceutical composition for use of claim 2, wherein the autoantibody is IgG, IgG1 or IgG2a.

5. The pharmaceutical composition for use of claim 1, wherein the composition comprises the N-ethyl-N-(4-fluorophenyl)-biguanide or a pharmaceutically acceptable salt thereof in the concentration ranging from 0.1 mM to 10 mM.

6. The pharmaceutical composition for use of claim 1, wherein the immune disease is selected from the group consisting of an autoimmune disease; an inflammatory disease; and a transplantation rejection disease.

7. The pharmaceutical composition for use of claim 6, wherein the autoimmune disease is selected from rheumatoid arthritis, Behcet's disease, polymyositis, autoimmune cytopenia, autoimmune myocarditis, atopic dermatitis, asthma, primary cirrhosis, dermatomyositis, Goodpasture's syndrome, autoimmune meningitis, Sjogren's syndrome, lupus, Addison's disease, alopecia areata, ankylosing myelitis, autoimmune hepatitis, autoimmune mumps, Crohn's disease, insulin-dependent diabetes, dystrophic epidermolysis bullosa, epididymitis, glomerulonephritis, Graves' disease, Guillain-Barre syndrome, Hashimoto's disease, hemolytic anemia, multiple sclerosis, myasthenia gravis, pemphigus vulgaris, psoriasis, rheumatic fever, sarcoidosis, scleroderma, spondyloarthropathy, thyroiditis, vasculitis, vitiligo, myxedema, pernicious anemia, mitochondria-related syndrome and ulcerative colitis.

8. The pharmaceutical composition for use of claim 6, wherein the transplantation rejection disease is graft versus host disease.

## Patentansprüche

1. Eine pharmazeutische Zusammensetzung für die Verwendung bei der Ermöglichung oder Verstärkung der Aktivität der regulatorischen T-Zellen und Reduzierung oder Hemmung der Aktivität der pathogenen Th17-Zellen in einer Immunkrankheit, umfassend N-Ethyl-N-(4-Fluorphenyl)-Biguanid oder ein pharmazeutisch zulässiges Salz davon als Wirkstoff.

2. Die pharmazeutische Zusammensetzung zur Verwendung nach Anspruch 1, wobei das N-Ethyl-N-(4-Fluorphenyl)-Biguanid oder ein pharmazeutisch zulässiges Salz davon die proinflammatorische Cytokin-Bildung weiter reduziert oder hemmt, die Autoantikörper-Produktion und die Abspaltung in Osteoklasten hemmt.

3. Die pharmazeutische Zusammensetzung für die Verwendung nach Anspruch 2, wobei das proinflammatorische Cytokin IL-17, IL-6, TNF-α, IFN-γ, MMP-9 oder STAT-3 ist.

4. Die pharmazeutische Zusammensetzung für die Verwendung nach Anspruch 2, wobei der Autoantikörper IgG, lgG1 oder lgG2a ist.

5. Die pharmazeutische Zusammensetzung für die Verwendung nach Anspruch 1, wobei die Zusammensetzung das N-Ethyl-N-(4-Fluorphenyl)-Biguanid oder ein pharmazeutisch zulässiges Salz davon in der Konzentration im Bereich von 0,1 mM bis 10 mM vorliegt.

6. Die pharmazeutische Zusammensetzung für die Verwendung nach Anspruch 1, wobei die Immunkrankheit ausgewählt ist aus der Gruppe bestehend aus einer Autoimmunkrankheit; einer entzündlichen Krankheit;
und einer Transplantationsabstoßungskrankheit.

7. Die pharmazeutische Zusammensetzung zur Verwendung nach Anspruch 6, wobei die Autoimmunkrankheit ausgewählt ist aus rheumatoider Arthritis, Behçet-Krankheit, Polymyositis, autoimmuner Zytopenie, autoimmuner Myocarditis, atopischem Ekzem, Asthma, primärer Zirrhose, Dermatomyositis, Goodpasture-Syndrom, autoimmuner Meningitis, Sjögren-Syndrom, Lupus, Nebennierenrindeninsuffizienz, Alopecia Areata, ankylosierender Myelitis, autoimmuner Hepatitis, autoimmunem Mumps, Morbus Crohn, insulin-abhängiger Diabetes, dystrophischer Epidermolysis bullosa, Epididymitis, Glomerulonephritis, Morbus Basedow, Guillain-Barre-Syndrom, Hashimoto-Krankheit, hämolytischer Anämie, multipler Sklerose, Myasthenia gravis, Pemphigus vulgaris, Psoriasis, rheumatischem Fieber, Sarcoidosis, Sclerodermie, Spondyloarthropathie, Thyroiditis, Vaskulitis, Vitiligo, Myxödem, perniziöser Anemie, Mitochondrien-bezogenes Syndrom und Colitis ulcerosa.

8. Die pharmazeutische Zusammensetzung für die Verwendung nach Anspruch 6, wobei die Transplantationsabstoßungskrankheit eine Transplantat-Wirt-Reaktion ist.

## Revendications

1. Composition pharmaceutique destinée à être utilisée pour faciliter ou augmenter l'activité de cellules T régulatrices et réduire ou inhiber l'activité de cellules Th17 pathogènes dans une maladie immunitaire, comprenant du N-éthyl-N-(4-fluorophényl)-biguanide ou un sel pharmaceutiquement acceptable de celui-ci en tant qu'ingrédient actif.

2. Composition pharmaceutique destinée à être utilisée dans la revendication 1, dans laquelle le N-éthyl-N-(4-fluorophényl)-biguanide ou un sel pharmaceutiquement acceptable de celui-ci réduit ou inhibe encore davantage la formation de cytokines pro-inflammatoires, inhibe la production d'auto-anticorps et inhibe la différenciation en ostéoclastes.

3. Composition pharmaceutique destinée à être utilisée dans la revendication 2, dans laquelle la cytokine pro-inflammatoire est IL-17, IL-6, TNF-α, IFN-γ, IFN-γ, MMP-9 ou STAT-3.

4. Composition pharmaceutique destinée à être utilisée dans la revendication 2, dans laquelle l'auto-anticorps est IgG, lgG1 ou lgG2a.

5. Composition pharmaceutique destinée à être utilisée dans la revendication 1, dans laquelle la composition comprend le N-éthyl-N-(4-fluorophényl)-biguanide ou un sel pharmaceutiquement acceptable de celui-ci à une concentration allant de 0,1 mmole à 10 mmole.

6. Composition pharmaceutique destinée à être utilisée dans la revendication 1, dans laquelle la maladie immunitaire est choisie dans le groupe constitué par une maladie auto-immune ; une maladie inflammatoire ;
et une maladie de rejet de transplantation.

7. Composition pharmaceutique destinée à être utilisée dans la revendication 6, dans laquelle la maladie auto-immune est choisie parmi la polyarthrite rhumatoïde, la maladie de Behcet, la polymyosite, la cytopénie auto-immune, la myocardite auto-immune, la dermatite atopique, l'asthme, la cirrhose primaire, la dermatomyosite, le syndrome de Goodpasture, la méningite auto-immune, le syndrome de Sjogren, le lupus, la maladie d'Addison, l'alopécie areata, la myélite ankylosante, l'hépatite auto-immune, les oreillons auto-immuns, la maladie de Crohn, le diabète insulinodépendant, l'épidermolyse dystrophique bulleuse, l'épididymite, la glomérulonéphrite, la maladie de Graves, le syndrome de Guillain-Barré, la maladie de Hashimoto, l'anémie hémolytique, la sclérose en plaques, la myasthénie gravis, la pemphigus vulgaris, le psoriasis, le rhumatisme articulaire aigu, la sarcoïdose, la sclérodermie, la spondylarthropathie, la thyroïdite, la vascularite, le vitiligo, le myxoedème, l'anémie pernicieuse, le syndrome lié à la mitochondrie et la colite ulcéreuse.

8. Composition pharmaceutique destinée à être utilisée selon la revendication 6, dans laquelle la maladie de rejet de transplantation est la maladie du greffon contre l'hôte.
